# EUROPEAN PATENT SPECIFICATION

(11) **EP 0 964 925 B1**
(45) Date of publication and mention of the grant of the patent: **25.04.2007**
(21) Application number: 97926586.5
(22) Date of filing: 16.05.1997
(51) Int. Cl.: C12N 15/12, C07K 14/47, C12N 5/10, C12Q 1/68, G01N 33/50, A61K 48/00, A61K 39/395, A61K 38/17, C07K 14/705

(54) **A BASAL CELL CARCINOMA TUMOR SUPPRESSOR GENE**
TUMORSUPRESSORGEN AUS BASALZELLKARZINOM
GENE SUPPRESSEUR DE TUMEURS D'UN CARCINOME DE CELLULES BASALES

(30) Priority: 17.05.1996 US 17906 P; 21.05.1996 AU PO001196; 07.06.1996 AU PO036396; 14.06.1996 US 19765 P; 16.05.1997 US 857636
(43) Date of publication of application: 22.12.1999
(73) Proprietor: THE GOVERNMENT OF THE UNITED STATES OF AMERICA as represented by the Secretary of the Department of Health and Human Services, Bethesda, Maryland 20892 (US)
(72) Inventor: DEAN, Michael, Frederick, MD 21702-1201 (US); HAHN, Heidi, Washington, DC (US); WICKING, Carol, St.Lucia (AU); CHRISTIANSEN, Jeffrey, Brisbane (AU); ZAPHIROPOULOS, Peter G., Huddinge (SE); GAILANI, Mae R., New Haven, CT (US); SHANLEY, Susan, Brisbane (AU); CHIDAMBARAM, Abirami, Frederick, MD 21702-1201 (US); VORECHOVSKY, Igor, Huddinge (SE); HOLMBERG, Erika, Huddinge (SE); UNDEN, Anne Birgitte, Huddinge (SE); GILLIES, Susan, St. Lucia (AU); NEGUS, Kylie, St. Lucia (AU); SMYTH, Ian, St. Lucia (AU); PRESSMAN, Carol, New Haven, CT (US); LEFFELL, David J., New Haven, CT 06520 (US); GERRARD, Bernard, Frederick, MD 21702-2101 (US); GOLDSTEIN, Alisa, Bethesda, MD 20852 (US); WAINWRIGHT, Brandon, St. Lucia (AU); TOFTGARD, Rune, Huddinge (SE); CHENEVIX-TRENCH, Georgia, Brisbane (AU); BALE, Allen E., New Haven, CT 06520 (US)
(74) Representative: OK pat AG
(86) International application number: PCT/US1997/008433
(87) International publication number: WO 1997/043414

(56) References cited:
- WO-A-96/11260
- GOODRICH, L.V. ET AL.: "Conservation of the Hedgehog/ patched signaling pathway from flies to mice: induction of a mouse patched gene by hedgehog" GENES & DEVELOPMENT, vol. 10, no. 3, 1 February 1996, pages 301-312, XP002048318
- GAILANI, M.R. ET AL. : "Developmental defects in Gorlin syndrome related to a putative tumor suppressor gene on chromosome 9" CELL, vol. 69, 3 April 1992, NA US, pages 111-117, XP002048319
- HAHN, H. ET AL.: "A mammalian patched homolog is expressed in target tissues of sonic hedgehog and maps to a region associated with developmental abnormalities" JOURNAL OF BIOLOGICAL CHEMISTRY. (MICROFILMS), vol. 271, no. 21, 24 May 1996, MD US, pages 12125-12128, XP002048320
- HAHN, H. ET AL.: "Mutations of the human homolog of Drosophila patched in the nevoid basal cell carcinoma syndrome" CELL, vol. 85, no. 6, 14 June 1996, NA US, pages 841-851, XP002048321

## Description

### BACKGROUND OF THE INVENTION

This invention pertains to the field of oncology. In particular, this invention pertains to the discovery of a tumor suppressor gene implicated in the etiology of nevoid basal cell carcinoma syndrome (NBCCS) and various cancers including basal cell carcinomas.

Many cancers are believed to result from a series of genetic alterations leading to progressive disordering of normal cellular growth mechanisms (Nowell (1976) *Science* 194:23, Foulds (1958) *J. Chronic Dis.* 8:2). In particular, the deletion or multiplication of copies of whole chromosomes or chromosomal segments, or specific regions of the genome are common (*see, e.g.,* Smith *et al.* (1991) *Breast Cancer Res. Treat.* 18: Suppl. 1: 5-14; van de Vijer & Nusse (1991) *Biochim. Biophys. Acta*. 1072: 33-50; Sato *et al*. (1990) *Cancer. Res.* 50: 7184-7189). In particular, the amplification and deletion of DNA sequences containing proto-oncogenes and tumor-suppressor genes, respectively, are frequently characteristic of tumorigenesis. Dutrillaux *et al*. (1990) *Cancer Genet. Cytogenet.* 49: 203-217.

One cancer-related syndrome that appears to have a strong genetic base is the nevoid basal cell carcinoma syndrome (NBCCS). The nevoid basal cell carcinoma syndrome, also known as Gorlin syndrome and the basal cell nevus syndrome, is an autosomal dominant disorder that predisposes to both cancer and developmental defects (Gorlin (1995) *Dermatologic Clinics* 13: 113-125). Its prevalence has been estimated at 1 per 56,000, and 1-2% of medulloblastomas and 0.5% of basal cell carcinomas (BCCs) are attributable to the syndrome (Springate (1986) *J. Pediatr. Surg.* 21: 908-910; Evans *et al.* (1991) *British J. Cancer.* 64: 959-961). In addition to basal cell carcinomas (BCCs) and medulloblastomas, NBCCS patients are also at an increased risk for ovarian fibromas, meningiomas, fibrosarcomas, rhabdomyosarcomas, cardiac fibromas and ovarian dermoids (Evans *et al*. (1991) *supra*., Evans *et al*. (1993) *J. Med Genet.* 30: 460-464; Gorlin (1995) *supra.*).

Non-neoplastic features including odontogenic keratocysts (which are more aggressive in the second and third decades of life), pathognomonic dyskeratotic pittina of the hands and feet, and progressive intracranial calcification (usually evident from the second decade) are very common. There is a broad range of skeletal defects (Gorlin (1995) *supra*.; Shanley *et al.* (1994) *Am. J. Med Genet.* 50: 282-290) including rib, vertebral and shoulder anomalies, pectus excavatum, immobile thumbs and polydactyly. Craniofacial and brain abnormalities include cleft palate, characteristic coarse faces, strabismus, dysgenesis of the corpus callosum macrocephaly and frontal bossing (Gorlin (1995) *supra.).* Generalized overgrowth (Bale *et al*. (1991) *Am. J. Med. Genet.* 40: 206-210) and acromegalic appearance are common, but growth hormone and IGF1 levels are not elevated.

Implications for the affected individual can be severe, predominantly due to the prolific basal cell carcinomas which can number more than 500 in a lifetime (Shanley *et al*. (1994) *supra*). Expression of many features of the syndrome is variable, but the severity tends to breed true within families (Anderson *et al*. (1967) *Am. J. Hum. Genet.,* 19: 12-22). This variation between families may reflect specific phenotypic effects of different mutations, modifier genes, or environmental factors (sunlight exposure is likely to play a role in the age of onset and incidence of basal cell carcinomas). One third to one half of patients have no affected relatives and are presumed to be the product of new germ cell mutations (Gorlin (1995) *supra.*). Unilateral and segmental NBCCS are attributed to somatic mutation in one cell of an early embryo (Gutierrez and Mora (1986) *J. Am. Acad Dermatol.* 15: 1023-1029).

The NBCCS syndrome was mapped to one or more genes at chromosome 9q22-31 (Gailani *et al.* (1992) *Cell* 69: 111-117; Reis *et al.* (1992) *Lancet* 339: 617; Farndon *et al.* (1992) *Lancet* 339: 581-2). In addition, it has been demonstrated that the same region is deleted in a high percentage of basal cell carcinomas and other tumors related to the disorder (Gailani *et al.* (1992) *supra*.) thus suggesting that the NBCCS gene functions as a tumor suppressor. Inactivation of NBCCS gene(s) may be a necessary if not sufficient event for the development of basal cell carcinomas (Shanley *et al*. (1995) *Hum. Mol. Genet*. 4: 129-133; Gallani *et al*. (1996) *J. Natl. Canc. Inst*. 88: 349-354).

Since the original mapping of the gene in 1992, linkage studies have narrowed the *NBCCS* region to a 4 cM Interval between *D95180* and *D95196* (Goldstein *et al*. (1994) *Am. J. Hum. Genet*. 54: 765-773; Wicking *et al*. (1994) *Genomics* 22: 505-511). Reported recombination involving an unaffected individual tentatively placed the gene proximal to *D9S287* (Farndon *et al*. (1994) *Genomics* 23: 486-489). The 9q22 region, however, is very gene rich and appeared to contain at least two tumor suppressor genes. In addition, Harshman *et al*. (1995) *Hum. Mol. Genet*. 4: 1259-1266, showed that different methods of Identifying cDNAs from a genomic region result in a surprisingly different array of candidate genes. Thus, prior to this Invention the specific NBCCS gene was unknown.

Invertebrate and vertebrate patched (ptc) genes are provided by WO 96/11260. According to the abstract, this document discloses patched genes including the mouse and human patched genes, as well as methods for isolation of related genes, where the genes may be of different species or in the same family. The abstract states that having the ability to regulate the expression of the patched (ptc) gene allows for the elucidation of embryonic development, cellular regulation associated with signal transduction by the patched gene disclosed in the reference, the Identification of agonist and antagonist to signal transduction, identification of ligands for binding to patched genes, isolation of the ligands, and assaying for levels of transcription and expression of the patched gene disclosed by the reference.

### SUMMARY OF THE INVENTION

This invention provides for a nucleic acid sequence (*e*.*g*., a cDNA) associated with nevoid basal cell carcinoma syndrome (NBCCS) and with various cancers including various sporadic basal cell carcinomas (BCCs). The NBCCS gene disclosed herein appears to be a tumor-suppressor gene and is a homologue of the *Drosophila patched (ptc)* gene. The human *NBCCS* gene is therefore also referred to herein as the human *PATCHED (PTC)* gene.

Absence, partial Inactivation (*e*.*g*., through haploinsufficiency or mutation), complete inactivation, or otherwise altered expression of the *NBCCS (PTC)* gene causes or creates a predisposition to NBCCS and/or to the onset of basal cell carcinomas. In one preferred embodiment, this invention provides an isolated human nucleic acid encoding a nevoid basal cell carcinoma syndrome (NBCCS) (*PTC*) protein, wherein said nucleic acid has the sequence set forth at nucleotides 442 to 4329 of SEQ ID NO: 1.

In another embodiment, this invention provides for vectors incorporating the above-described nucleic acid. The vectors preferably include the above-described nucleic acid operably linked (under the control of) a promoter; either constitutive or inducible. The vector can also include an initiation and a termination codon.

This invention also provides for an isolated human NBCCS (*PTC*) polypeptide, said polypeptide encoded by SEQ ID NO: 1.

In another embodiment, this invention provides for pharmacological compositions comprising a pharmaceutically acceptable carrier and a molecule selected from the group consisting of an vector encoding SEQ ID NO:60 or a polypeptide having the amino acid sequence of SEQ ID NO:60.

Finally, this invention also provides therapeutic methods. These include a methods of treating basal cell carcinoma and/or nevoid basal cell carcinoma syndrome and/or solar keratoses in a mammal. The methods can involve transfecting cells of the mammal with a vector expressing a nevoid basal cell carcinoma syndrome (NBCCS) polypeptide such that the cells express a functional NBCCS polypeptide as described herein. The transfection can be *in vivo* or *ex vivo. Ex vivo* transfection is preferably followed by re-infusion of the cells back into the organism as described herein. Other methods involve administering to the mammal a therapeutically effective dose of a composition comprising a NBCCS (*PTC*) polypeptide and a pharmacological excipient as described herein. The methods are preferably performed on mammals such as mice, rats, rabbits, sheep, goats, pigs, more preferably on primates including human patients.

### Definitions

The terms "isolated" "purified" or "biologically pure" refer to material which is substantially or essentially free from components which normally accompany it as found in its native state.

The term "nucleic acid" refers to a deoxyribonucleotide or ribonucleotide polymer in either single- or double-stranded form, and unless otherwise limited, encompasses known analogs of natural nucleotides that can function in a similar manner as naturally occurring nucleotides.

The terms "polypeptide", "peptide" and "protein" are used interchangeably herein to refer to a polymer of amino acid residues. The terms apply to amino acid polymers in which one or more amino acid residue is an artificial chemical analogue of a corresponding naturally occurring amino acid, as well as to naturally occurring amino acid polymers.

A "label" is a composition detectable by spectroscopic, photochemical, biochemical, immunochemical, or chemical means. For example, useful labels include ³²P, fluorescent dyes, electron-dense reagents, enzymes (*e*.*g*., as commonly used in an ELISA), biotin, dioxigenin, or haptens and proteins for which antisera or monoclonal antibodies are available (*e.g.*, the peptide of SEQ ID NO 1 can be made detectable, *e.g.*, by incorporating a radio-label into the peptide, and used to detect antibodies specifically reactive with the peptide).

The term "recombinant" when used with reference to a cell, or nucleic acid, or vector, indicates that the cell, or nucleic acid, or vector, has been modified by the introduction of a heterologous nucleic acid or the alteration of a native nucleic acid, or that the cell is derived from a cell so modified. Thus, for example, recombinant cells express genes that are not found within the native (non-recombinant) form of the cell or express native genes that are otherwise abnormally expressed, under expressed or not expressed at all.

The term "identical" in the context of two nucleic acids or polypeptide sequences refers to the residues in the two sequences which are the same when aligned for maximum correspondence. Optimal alignment of sequences for comparison can be conducted, *e.g.,* by the local homology algorithm of Smith and Waterman (1981) *Adv. Appl. Math*. 2: 482, by the homology alignment algorithm of Needleman and Wunsch (1970) *J. Mol. Biol*. 48:443, by the search for similarity method of Pearson and Lipman (1988) *Proc. Natl. Acad Sci. USA* 85: 2444, by computerized implementations of these algorithms (GAP, BESTFIT, FASTA, and TFASTA in the Wisconsin Genetics Software Package, Genetics Computer Group, 575 Science Dr., Madison, WI), or by inspection.

An additional algorithm that is suitable for determining sequence similarity is the BLAST algorithm, which is described in Altschul *et al.* (1990) *J. Mol. Biol.* 215: 403-410. Software for performing BLAST analyses is publicly available through the National Center for Biotechnology Information (http://www.ncbi.nlm.nih.gov/). This algorithm involves first identifying high scoring sequence pairs (HSPs) by identifying short words of length W in the query sequence that either match or satisfy some positive-valued threshold score T when aligned with a word of the same length in a database sequence. T is referred to as the neighborhood word score threshold (Altschul *et al, supra*.). These initial neighborhood word hits act as seeds for initiating searches to find longer HSPs containing them. The word hits are extended in both directions along each sequence for as far as the cumulative alignment score can be increased. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T and X determine the sensitivity and speed of the alignment. The BLAST program uses as defaults a word length (W) of 11, the BLOSUM62 scoring matrix (*see* Henikoff and Henikoff (1992) *Proc. Natl. Acad. Sci. USA* 89: 10915-10919) alignments (B) of 50, expectation (E) of 10, M=5, N=-4, and a comparison of both strands.

The BLAST algorithm performs a statistical analysis of the similarity between two sequences; *see, e*.*g*., Karlin and Altschul (1993) *Proc. Nat'l. Acad Sci. USA* 90: 5873-5787. One measure of similarity provided by the BLAST algorithm is the smallest sum probability (P(N)), which provides an indication of the probability by which a match between two nucleotide or amino acid sequences would occur by chance. For example, a nucleic acid is considered similar to an NBCCS gene or cDNA if the smallest sum probability in a comparison of the test nucleic acid to an NBCCS nucleic acid (*e*.*g*., SEQ ID Nos: 1, 58, or 59) is less than about 1, preferably less than about 0.1, more preferably less than about 0.01, and most preferably less than about 0.001.

The term "substantial identity" or "substantial similarity" in the context of a polypeptide indicates that a polypeptide comprises a sequence with at least 70% sequence identity to a reference sequence, or preferably 80%, or more preferably 85% sequence identity to the reference sequence, or most preferably 90% identity over a comparison window of about 10-20 amino acid residues. An indication that two polypeptide sequences are substantially identical is that one peptide is immunologically reactive with antibodies raised against the second peptide. Thus, a polypeptide is substantially identical to a second polypeptide, for example, where the two peptides differ only by a conservative substitution.

An indication that two nucleic acid sequences are substantially identical is that the polypeptide which the first nucleic acid encodes is immunologically cross reactive with the polypeptide encoded by the second nucleic acid.

Another indication that two nucleic acid sequences are substantially identical is that the two molecules hybridize to each other under stringent conditions.

"Bind(s) substantially" refers to complementary hybridization between a probe nucleic acid and a target nucleic acid and embraces minor mismatches that can be accommodated by reducing the stringency of the hybridization media to achieve the desired detection of the target polynucleotide sequence.

The phrase "hybridizing specifically to", refers to the binding, duplexing, or hybridizing of a molecule only to a particular nucleotide sequence under stringent conditions when that sequence is present in a complex mixture (*e*.*g*., total cellular) DNA or RNA. The term "stringent conditions" refers to conditions under which a probe will hybridize to its target subsequence, but to no other sequences. Stringent conditions are sequence-dependent and will be different in different circumstances. Longer sequences hybridize specifically at higher temperatures. Generally, stringent conditions are selected to be about 5°C lower than the thermal melting point (Tm) for the specific sequence at a defined ionic strength and pH. The Tm is the temperature (under defined ionic strength, pH, and nucleic acid concentration) at which 50% of the probes complementary to the target sequence hybridize to the target sequence at equilibrium. (As the target sequences are generally present in excess, at Tm, 50% of the probes are occupied at equilibrium). Typically, stringent conditions will be those in which the salt concentration is less than about 1.0 M Na ion, typically about 0.01 to 1.0 M Na ion concentration (or other salts) at pH 7.0 to 8.3 and the temperature is at least about 30°C for short probes (*e.g.*, 10 to 50 nucleotides) and at least about 60°C for long probes (*e*.*g*., greater than 50 nucleotides). Stringent conditions may also be achieved with the addition of destabilizing agents such as formamide.

The phrases "specifically binds to a protein" or "specifically immunoreactive with", when referring to an antibody refers to a binding reaction which is determinative of the presence of the protein in the presence of a heterogeneous population of proteins and other biologics. Thus, under designated immunoassay conditions, the specified antibodies bind preferentially to a particular protein and do not bind in a significant amount to other proteins present in the sample. Specific binding to a protein under such conditions requires an antibody that is selected for its specificity for a particular protein. A variety of immunoassay formats may be used to select antibodies specifically immunoreactive with a particular protein. For example, solid-phase ELISA immunoassays are routinely used to select monoclonal antibodies specifically immunoreactive with a protein. See Harlow and Lane (1988) *Antibodies, A Laboratory Manual,* Cold Spring Harbor Publications, New York, for a description of immunoassay formats and conditions that can be used to determine specific immunoreactivity.

A "conservative substitution", when describing a protein refers to a change in the amino acid composition of the protein that does not substantially alter the protein's activity. Thus, "conservatively modified variations" of a particular amino acid sequence refers to amino acid substitutions of those amino acids that are not critical for protein activity or substitution of amino acids with other amino acids having similar properties (*e.g.*, acidic, basic, positively or negatively charged, polar or non-polar, *etc.*) such that the substitutions of even critical amino acids do not substantially alter activity. Conservative substitution tables providing functionally similar amino acids are well known in the art. The following six groups each contain amino acids that are conservative substitutions for one another:
1) Alanine (A), Serine (S), Threonine (T);
2) Aspartic acid (D), Glutamic acid (E);
3) Asparagine (N), Glutamine (Q);
4) Arginine (R), Lysine (K);
5) Isoleucine (I), Leucine (L), Methionine (M), Valine (V); and
6) Phenylalanine (F), Tyrosine (Y), Tryptophan (W).
See also, Creighton (1984) *Proteins,* W.H. Freeman and Company. In addition, individual substitutions, deletions or additions which alter, add or delete a single amino acid or a small percentage of amino acids in an encoded sequence are also "conservatively modified variations" .

The terms human "*PTC*" or human "NBCCS gene or cDNA" are used interchangeably to refer to the human homologue of the *Drosophila patched* (*ptc*) gene disclosed herein. As explained below, the human *PTC* gene is a tumor suppressor gene also involved in the etiology of nevoid basal carcinoma cell syndrome.

A "gene product", as used herein, refers to a nucleic acid whose presence, absence, quantity, or nucleic acid sequence is indicative of a presence, absence, quantity, or nucleic acid composition of the gene. Gene products thus include, but are not limited to, an mRNA transcript, a cDNA reverse transcribed from an mRNA, an RNA transcribed from that cDNA, a DNA amplified from the cDNA, an RNA transcribed from the amplified DNA or subsequences of any of these nucleic acids. Polypeptides expressed by the gene or subsequences thereof are also gene products. The particular type of gene product will be evident from the context of the usage of the term.

An "abnormal *PTC* (or NBCCS) gene or cDNA" refers to a NBCCS gene or cDNA that encodes a non-functional NBCCS polypeptide, or an NBCCS polypeptide of substantially reduced functionality. Non-functional, or reduced functionality, NBCCS polypeptides are characterized by a predisposition (*i.e.,* an increased likelihood as compared to the "normal" population) for, or the onset of, nevoid basal cell carcinoma syndrome. Similarly, "abnormal *PTC* (or NBCCS) gene product" refers to a nucleic acid encoding a non-functional or reduced functionality NBCCS polypeptide or the non-functional or reduced functionality NBCCS polypeptide itself. Abnormal *NBCCS (PTC)* genes or gene products include, for example, NBCCS genes or subsequences altered by mutations (*e.g.* insertions, deletions, point mutations, *etc.*), splicing errors, premature termination codons, missing initiators, *etc*. Abnormal NBCCS polypeptides include polypeptides expressed by abnormal *NBCCS* genes or nucleic acid gene products or subsequences thereof. Abnormal expression of *NBCCS* genes includes underexpression (as compared to the "normal" healthy population) of *NBCCS e*.*g*., through partial or complete inactivation, haploinsufficiency, *etc.*

### BRIEF DESCRIPTION OF THE DRAWINGS

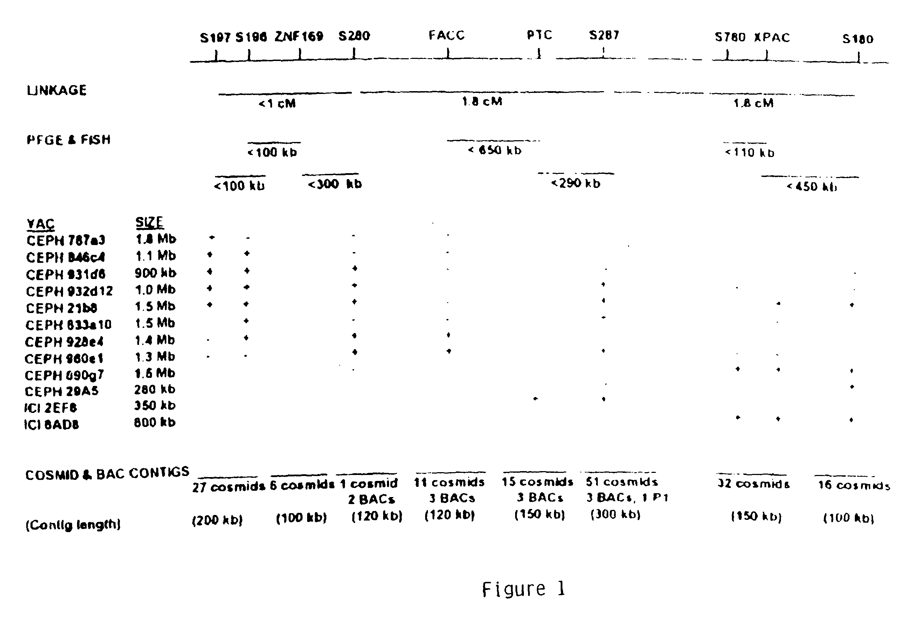

Figure 1 shows an integrated framework map of the NBCCS region. Both linkage and tumor deletion studies place *NBCCS* between *D9S196* and *D9S180* but are conflicting, with regard to whether the gene lies proximal or distal to *D9S287.* The order of six polymorphic markers, *D9S197, D9S196-D9S280-FACC-D9S287-D9S180,* is derived from genetic linkage data (Farndon *et al*., 1994; Pericak-Vance *et al.,* 1995). *D9S196* and *D9S197* show no measurable recombination. Pulsed-field gel electrophoresis (PFGE) and FISH give a minimum distance of 2 Mb between *D9S196* and *D9S180*. Key information about YAC, BAC, and cosmid contigs in the *NBCCS* region is shown. In total, 22 overlapping YACs and more than 800 cosmids were isolated from this region. BAC and cosmid contigs covering more than 1 .2 Mb have been submitted to the Genome Data Base.

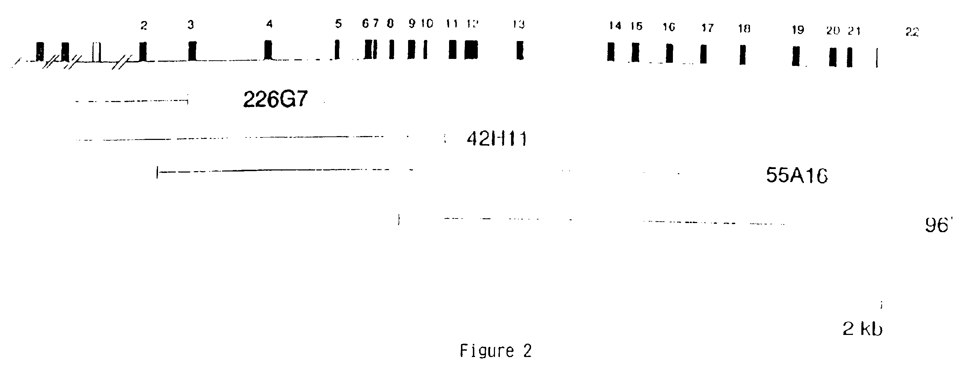

Figure 2 shows a map of the *PTC* locus. The gene lies on 4 overlapping cosmids including 226G7, 42H11, 55A16, and 96F9 (LL09NCO1, 96-well coordinates). The coding exons of the gene are shown as filled boxes and non-coding (untranslated) exons as open boxes. Splice variants of the 5' non-coding, region of the gene indicate at least two alternate first exons and possibly a third alternate exon (see Example 2).

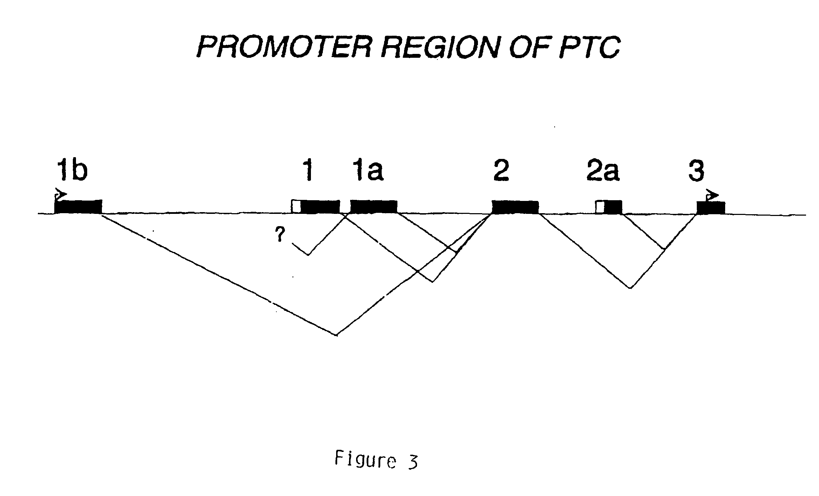

Figure 3 provides a map of the promoter region of the NBCCS (*PTC*) gene.

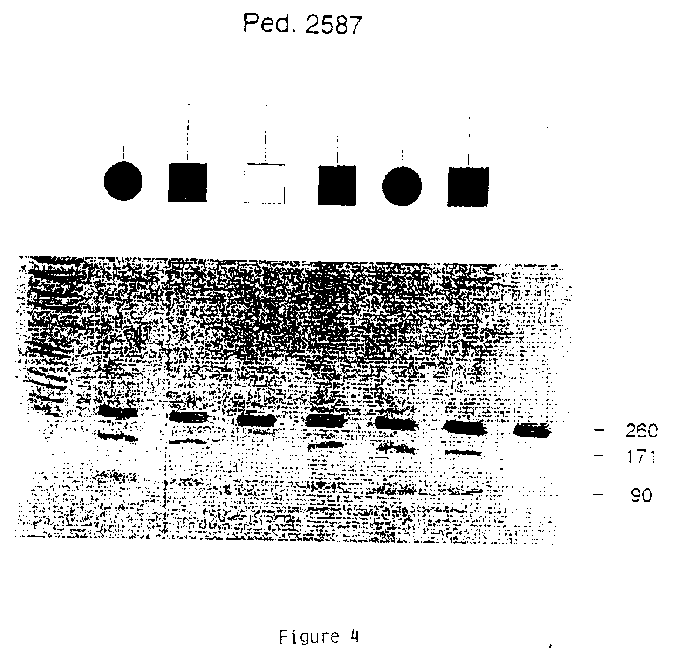

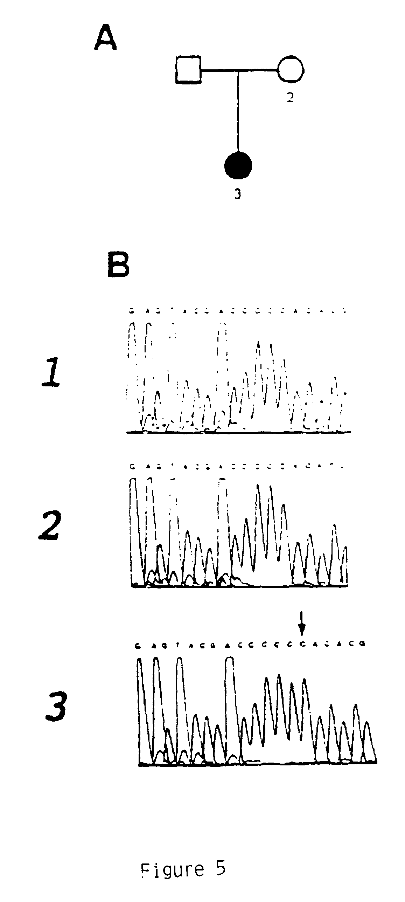

Figure 4 illustrates segregation of a premature termination mutation of *PTC* in an NBCCS pedigree. The C1081T (Q210X) mutation segregating in this kindred creates a Bfal site. PCR with flanking primers produces a 260 bp product, which features of NBCCS. (Figure 5B) The patient was heterozygous for a single stranded conformation polymorphism (SSCP) variant in Exon 12 that was not present in her parents, and sequencing of a PCR product from genomic DNA showed two sequences out of frame following base 2000. The abnormal conformer was sequenced (SEQ ID NO:56) and contained a 1 bp insertion resulting in a premature stop nine amino acids downstream. Sequences of PCR products from both parents were normal.

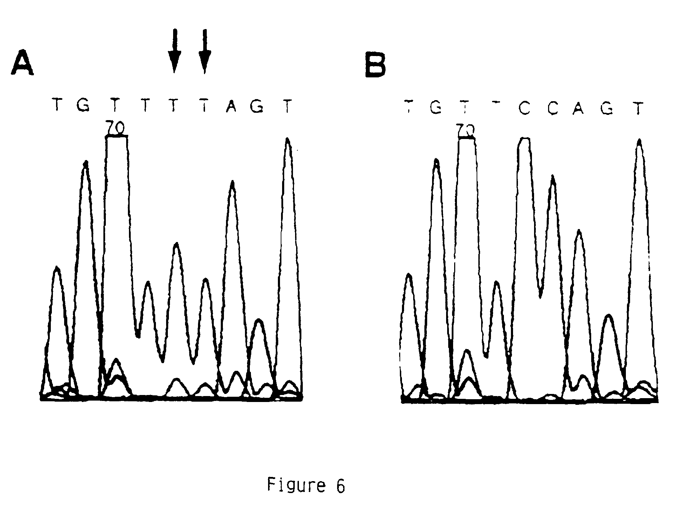

Figure 6 shows an ultraviolet B-induced mutation of *PTC* in a sporadic basal cell carcinoma. (Figure 6A) CC to TT mutation in the remaining allele with allelic loss of the *NBCCS* region. This DNA alteration, which results in a premature stop, is typical of ultraviolet B mutagenesis. (Figure 6B) Constitutional DNA from the patient has a normal sequence.

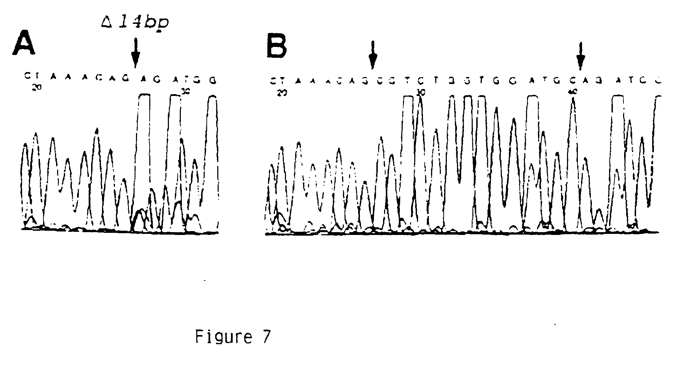

Figure 7 shows a *PTC* deletion in a sporadic BCC. (Figure 7A) A 14 bp deletion in the remaining allele of a tumor with allelic loss of the *NBCCS* region. Despite the fact that this tumor was removed from the nose, a highly sunlight-exposed area, the mutation cannot be related specifically to ultraviolet radiation. (Figure 7B) Constitutional DNA had the normal sequence.

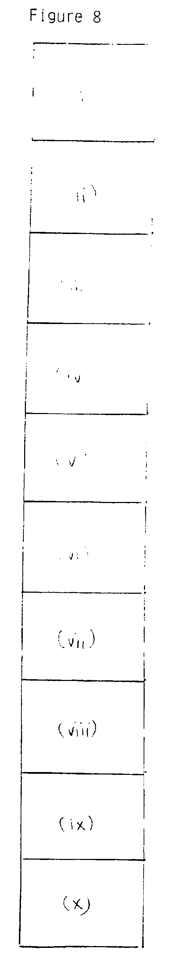

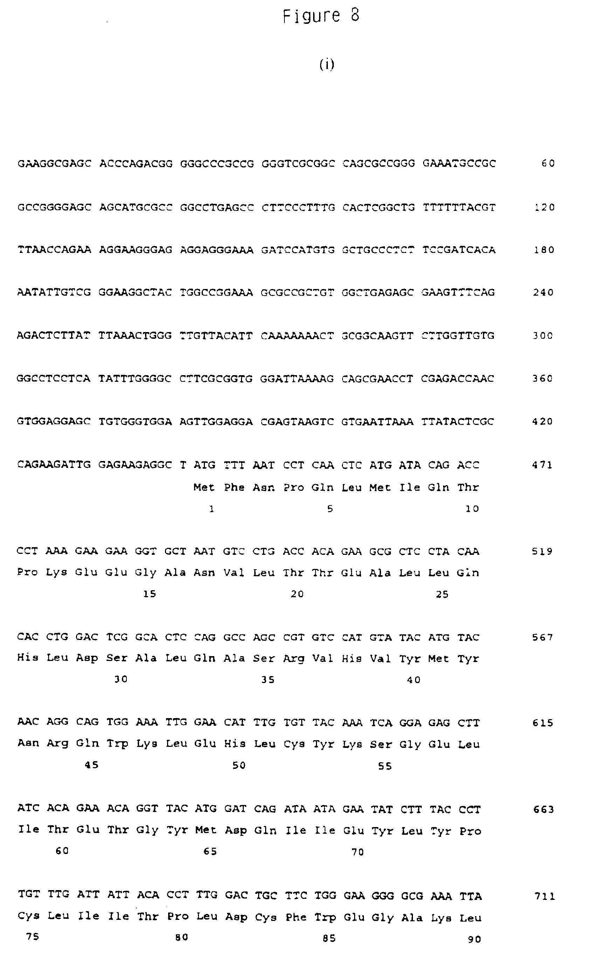

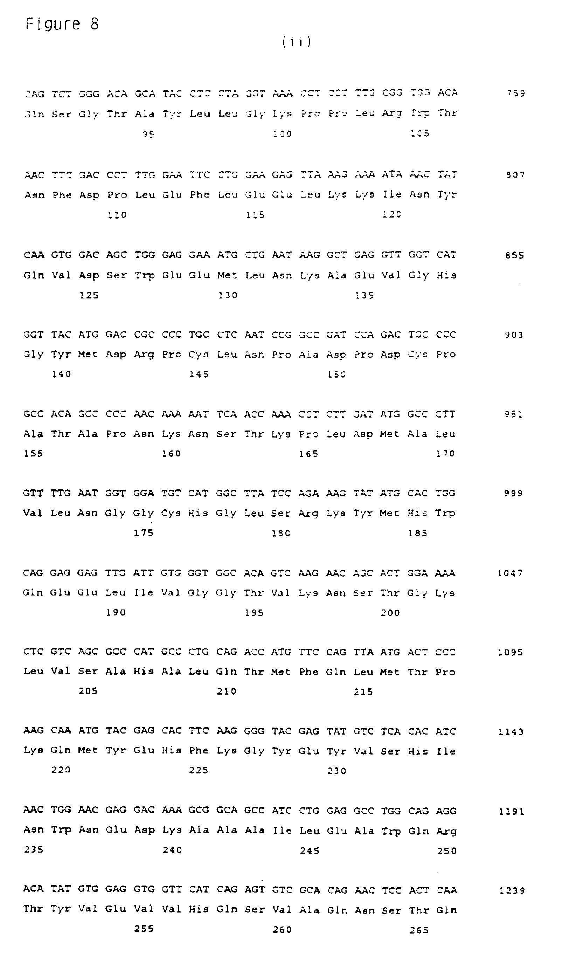

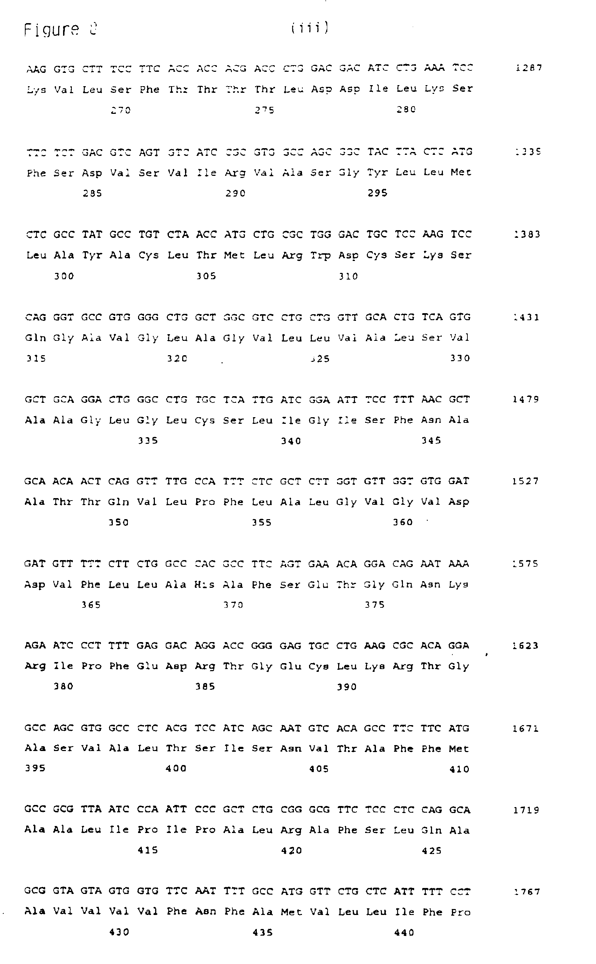

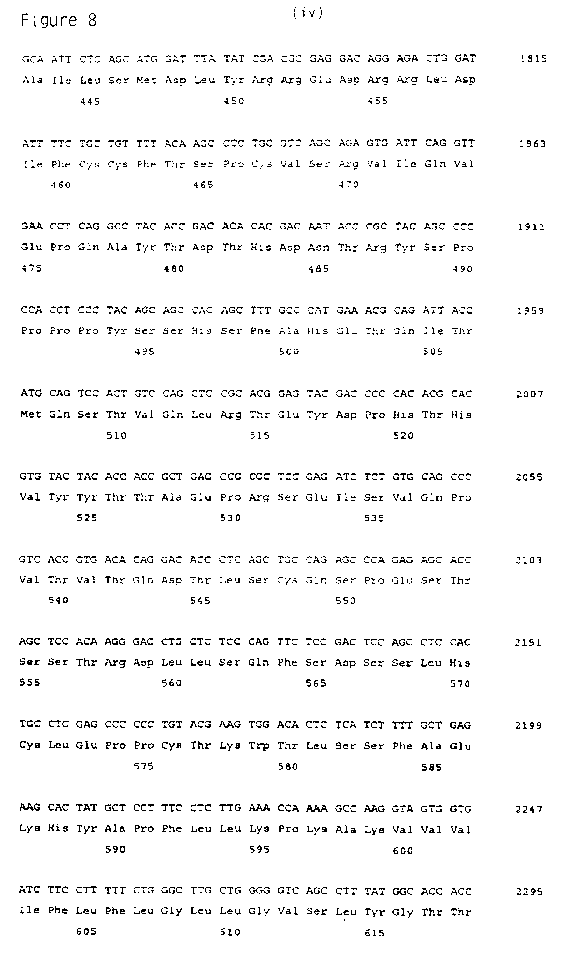

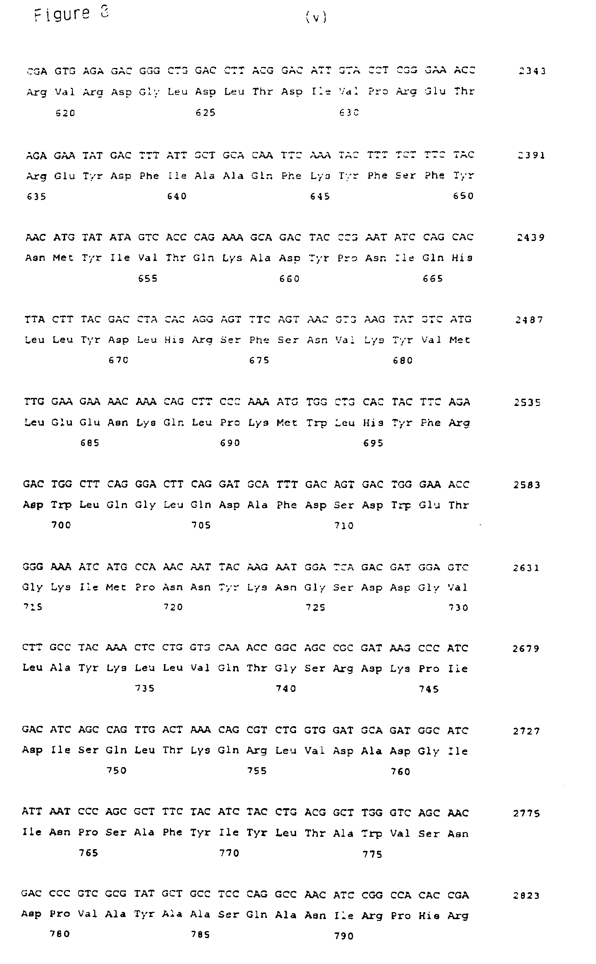

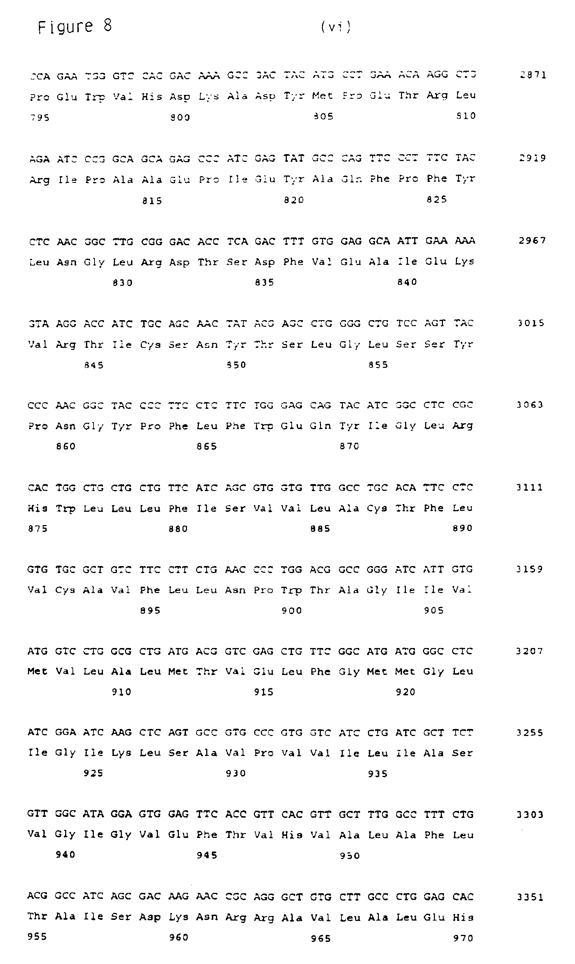

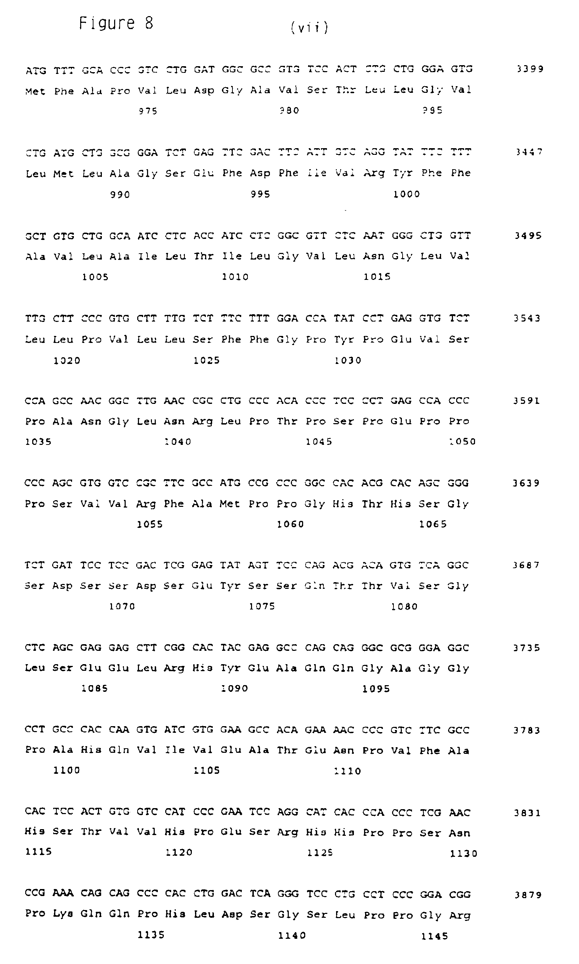

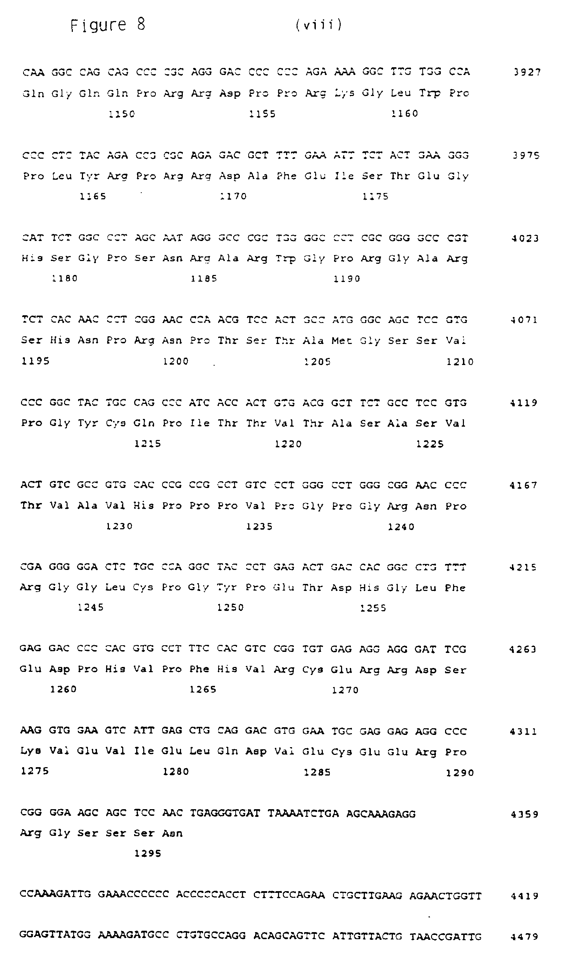

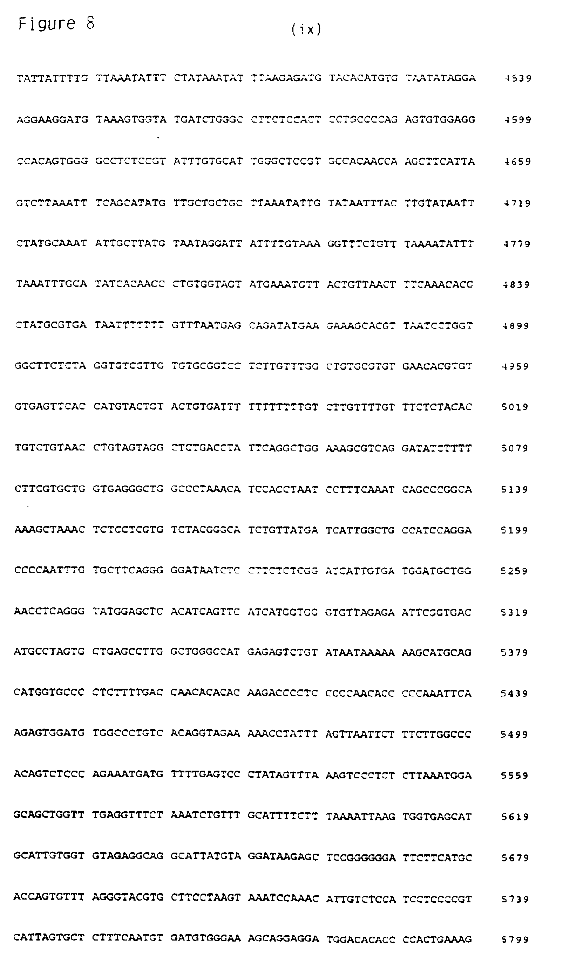

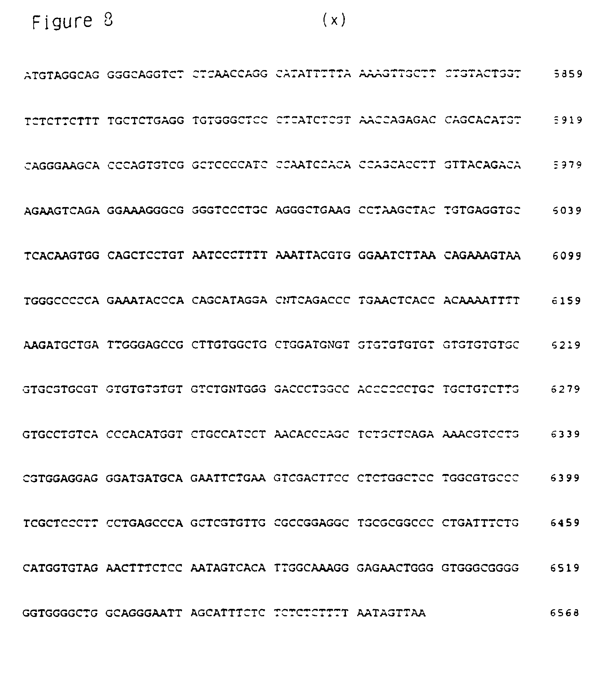

Figure 8 shows the nucleotide sequence (SEQ ID NO:1) of the human *PTC* cDNA (Genbank Accession No. U43148). The sequence of *PTC* is shown, including the open reading frame and flanking 5' and 3' sequences. The corresponding amino acid sequence is presented in SEQ ID NO:60.

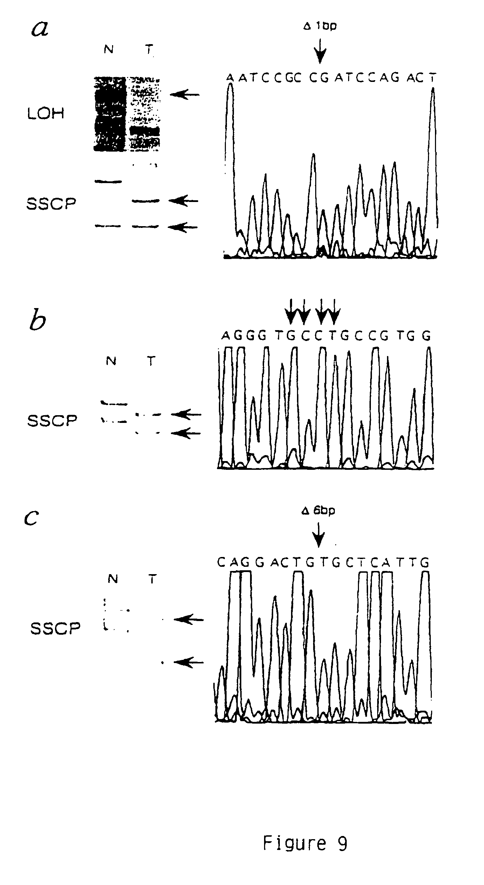

Figure 9 shows mutations in the human *PTC* gene in DNA obtained from desmoplastic medulloblastomas D322, D292 and D86. Figure 9A: microsatellite D9S302 shows loss of heterozygosity in tumor D322. In this tumor, the remaining allele of exon 6 exhibits an altered mobility. DNA sequencing shows a single base pair deletion in the tumor DNA which results in a frameshift and resulting truncation of the PTC protein. Figure 9B shows SSCP variants in exon 10 in tumor D292 without allelic loss of chromosome 9q. Sequencing of the altered allele shows a four base pair insertion at position 1393. Figure 9C shows the sequencing of exon 10 in tumor D86. This tumor, which has LOH, has a six base pair in-frame deletion at position 1444 (CTG GGC). This leads to a deletion of two amino acids (glycine and leucine) in transmembrane region 3.

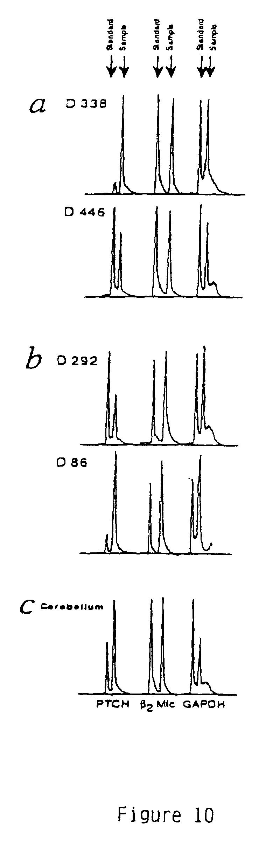

Figure 10 shows *PTC* mRNA levels as determined using a semi-quantitative RT-PCR approach as described in Example 5. 250 ng of RNA in 10 µl was reverse transcribed in the presence of 40 pg of each of the standard RNAs with internal deletions for *PTC,* β₂-microglobin and *GAPDH.* The cDNAs were then amplified with primers for *PTC* and the housekeeping genes. The products were separated and quantitated on an ABI 373A sequencer. The expression level of the three genes were determined as the ratio of signals of the sample (right peaks) to the specific standards (left peaks). Figure 10A: *PTC* mRNA expression in two representative tumors of the classical variant of MB; Figure 10B: expression in desmoplastic MBs; Figure 10C: expression in adult human cerebellum.

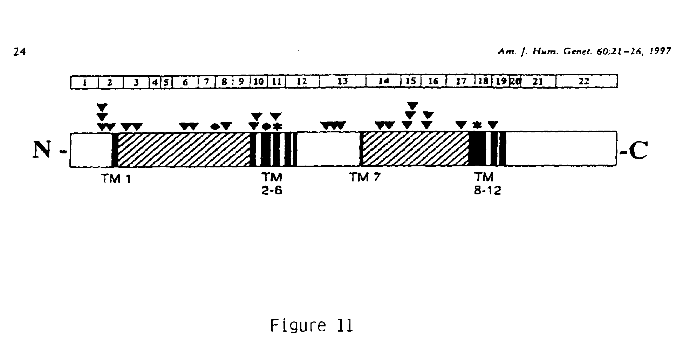

Figure 11 is a schematic diagram of the PTC protein with the location of germ line mutations indicated. The putative transmembrane domains are designated "TM1" to "TM12"; the hatched boxes represent the two putative extracellular loops. An asterisk (*) denotes a missense; a triangle (▼) denotes a nonsense or frameshift; and a diamond (◆) denotes putative splicing variants. Open boxes above correspond to exons encoding relevant domains.

### DETAILED DESCRIPTION

This invention pertains to the discovery of a tumor suppressor gene associated with the etiology of nevoid basal cell carcinoma syndrome. In addition, this invention pertains to the discovery that various cancers, including sporadic basal cell carcinomas (BCCs) can arise with somatic loss of both copies of the same gene.

Nevoid basal cell carcinoma syndrome, also known as Gorlin syndrome and the basal cell nevus syndrome, is an autosomal dominant disorder that predisposes to both cancer and developmental defects (Gorlin (1995) *Dermatologic Clinics,* 13: 113-125). Its prevalence has been estimated at 1 per 56,000, and 1-2% of medulloblastomas and 0.5% of basal cell carcinomas (BCCS) are attributable to the syndrome (Springate (1986) *J. Pediatr. Surg.* 21: 908-910; Evans *et al*. (1991) *British J. Cancer*., 64: 959-961). In addition to basal cell carcinomas (BCCs) and medulloblastomas, NBCCS patients are also at an increased risk for ovarian fibromas, meningiomas, fibrosarcomas, rhabdomyosarcomas, cardiac fibromas and ovarian dermoids (Evans *et al.* (1991) *supra*., Evans *et al.* (1993) *J. Med. Genet. 30:* 460-464; Gorlin (1995) *supra.).*

Implications for the affected individual can be severe, predominantly due to the prolific basal cell carcinomas which can number more than 500 in a lifetime (Shanley *et al.* (1994) supra). Expression of many features of the syndrome is variable, but the severity tends to breed true within families (Anderson *et al*. (1967) *Am. J. Hum. Genet.,* 19: 12-22). This variation between families may reflect specific phenotypic effects of different mutations, modifier genes, or environmental factors (sunlight exposure is likely to play a role in the age of onset and incidence of basal cell carcinomas). One third to one half of patients have no affected relatives and are presumed to be the product of new germ cell mutations (Gorlin (1995) supra.). Unilateral and segmental NBCCS are attributed to somatic mutation in one cell of an early embryo (Gutierrez and Mora, (1986) *supra*.)

### I. Uses of the NBCCS (PTC) cDNA.

As indicated above, the *NBCCS* gene of this invention is a tumor suppressor gene. Defects in the expression of this gene are associated with the onset of various cancers, particularly with sporadic basal cell carcinoma in somatic cells. Heritable defects in the expression of the NBCCS gene are a causal factor in the etiology of NBCCS and its attendant developmental abnormalities (see example 2, below).

While basal cell carcinomas and many features of NBCCS are believed to be due to homozygous inactivation of *PTC* generalized or symmetric features such as overgrowth, macrocephaly, and facial dysmorphology are believed to be due to haploinsufficiency or other mechanisms of partial gene inactivation.

Clearly, detection of defective NBCCS (*PTC*) gene expression is of clinical value. The presence of an *NBCCS* (*PTC*) gene, cDNA, mRNA, protein, or subsequence of the gene, cDNA, or protein in a biological sample is useful, *e.g.,* as a marker to assess *in vivo* and/or *in situ* RNA transcription and/or translation, in cancer diagnostics (as in the detection or verification of basal cell carcinoma), in prophylaxis for NBCCS or BCC as an indication of a heritable predilection for NBCCS or BCC, or in DNA forensic analysis such as DNA fingerprinting. Full-length *NBCCS* cDNA, individual exons, or subsequences thereof are also useful as probes (particularly when labeled) for the detection of the presence or absence and/or quantitation of normal or abnormal (*e*.*g*., truncated or mutated) *NBCCS* (*PTC*) DNA or RNA in a biological sample. The labeled probes can also be useful as in fluorescent karyotyping analysis as markers of the *NBCCS* gene. Because the *NBCCS* cDNA or subsequences thereof is shown herein to map to human chromosome 9q22.3, one of skill can use the gene, cDNA, or subsequences, as a probe to assess whether there are any gross chromosomal abnormalities in this region of chromosome 9. This is useful, for instance, in *in utero* screening of a fetus to monitor for the presence of chromosomal abnormalities in particular for a predilection of NBCCS or basal cell carcinomas.

Similarly, the proteins encoded by the NBCCS cDNA can be used as diagnostic markers for NBCCS and/or basal cell carcinomas. The proteins or subsequences thereof can also be used as antigens for raising anti-NBCCS protein antibodies. The antibodies are useful for immunoassays for the detection of normal or abnormal expression of NBCCS proteins, and for the isolation of NBCCS polypeptides (as with affinity chromatography).

Vectors encoding the NBCCS proteins are useful for expressing those proteins to provide immunogens for antibody production. Vectors encoding the NBCCS proteins are also useful for transforming cells *in vitro* or *in vivo* to express NBCCS proteins. *In vivo* transformation of cells to express heterologous NBCCS genes can be used to offset deficient expression of the NBCCS protein.

Cells and/or tissues expressing the NBCCS (*PTC*) gene may be used to monitor expression levels of NBCCS polypeptides in a wide variety of contexts. For example, where the effects of a drug on NBCCS expression is to be determined the drug will be administered to the transformed (to express NBCCS) organism, tissue, or cell. Expression levels, or expression products will be assayed as described below and the results compared to results from to organisms, tissues, or cells similarly treated, but without the drug being tested.

### II. The NBCCS (PTC) gene.

### A) The human PTC gene.

SEQ ID NO:1 provides both nucleic acid and polypeptide sequence listings for the human *PTC* cDNA of this invention. The sequence of human *PTC,* as shown, consists of an open reading frame of 3888 nucleotides flanked by 441 and 2240 nucleotides on the 5' end and on the 3' end, respectively (SEQ ID NO: 1, Fig. 8). The open reading frame of human *PTC* cDNA encodes for a putative protein of 1296 amino acids. The open reading frame starts with an ATG codon that has a moderate match for the translational start consensus sequence in vertebrates (GAGGCTATGT (SEQ ID NO: 6) in *PTC* versus GCCGCCATGG (SEQ ID NO: 7) (Kozak (1991) *J. Biol. Chem.* 266: 19867-19870)). This codon codes for the first amino acid of one human form of the *PTC* protein consisting of 1296 amino acids with a relative molecular weight (Mᵣ) of 131 x 10³. It shows 61% sequence identity to its *Drosophila* counterpart. The open reading frame extends an additional 354 nucleotides upstream of the ATG codon (starting at base pair 88 of the sequence shown in Figure 8). The 3' untranslated region contains a canonical polyadenylation signal (AATAAA (SEQ ID NO: 8)) as well as mRNA destabilizing (ATTTA (SEQ ID NO: 9)) motifs. These are localized 1401 nucleotides and 547, 743, and 1515 nucleotides after the termination codon, respectively. A second human *PTC* protein contains an open reading frame that continues right through to the 5' end, and may be initiated by upstream sequences.

### B) Isolation of cDNA and/or probes.

The nucleic acids (*e.g., NBCCS* cDNA, or subsequences (probes)) of the present invention are cloned, or amplified by *in vitro* methods, such as the polymerase chain reaction (PCR), the ligase chain reaction (LCR), the transcription-based amplification system (TAS), the self-sustained sequence replication system (SSR). A wide variety of cloning and *in vitro* amplification methodologies are well-known to persons of skill. Examples of these techniques and instructions sufficient to direct persons of skill through many cloning exercises are found in Berger and Kimmel, *Guide to Molecular Cloning Techniques, Methods in Enzymology* 152 Academic Press, Inc., San Diego, CA (Berger); Sambrook *et al.* (1989) *Molecular Cloning - A Laboratory Manual* (2nd ed.) Vol. 1-3, Cold Spring Harbor Laboratory, Cold Spring Harbor Press, NY, (Sambrook *et al*.); *Current Protocols in Molecular Biology,* F.M. Ausubel *et al.,* eds., Current Protocols, a joint venture between Greene Publishing Associates, Inc. and John Wiley & Sons, Inc., (1994 Supplement) (Ausubel); Cashion et al., U.S. patent number 5,017,478; and Carr, European Patent No. 0,246,864. Examples of techniques sufficient to direct persons of skill through *in vitro* amplification methods are found in Berger, Sambrook, and Ausubel, as well as Mullis *et al.,* (1987) U.S. Patent No. 4,683,202; *PCR Protocols A Guide to Methods and Applications* (Innis et al. eds) Academic Press Inc. San Diego, CA (1990) (Innis); Arnheim & Levinson (October 1, 1990) *C&EN* 36-47; *The Journal Of NIH Research* (1991) 3: 81-94; (Kwoh *et al.* (1989) *Proc. Natl. Acad. Sci. USA* 86: 1173; Guatelli *et al.* (1990) *Proc. Natl. Acad Sci. USA* 87, 1874; Lomell *et al*. (1989) *J. Clin. Chem.,* 35: 1826; Landegren *et al.,* (1988) *Science,* 241: 1077-1080; Van Brunt (1990) *Biotechnology*, 8: 291-294; Wu and Wallace, (1989) *Gene,* 4: 560; and Barringer *et al.* (1990) *Gene,* 89: 117.

In one preferred embodiment, the human *NBCCS* (*PTC*) cDNA can be isolated by routine cloning methods. The cDNA sequence provided in SEQ ID NO: 1 can be used to provide probes that specifically hybridize to the *NBCCS* gene, in a genomic DNA sample, or to the *NBCCS* mRNA, in a total RNA sample (*e*.*g*., in a Southern blot). Once the target *NBCCS* nucleic acid is identified (*e.g.*, in a Southern blot), it can be isolated according to standard methods known to those of skill in the art (*see, e*.*g*., Sambrook *et al.* (1989) *Molecular Cloning: A Laboratory Manual, 2nd Ed, Vols. 1-3*, Cold Spring Harbor Laboratory; Berger and Kimmel (1987) *Methods in Enzymology, Vol. 152: Guide to Molecular Cloning Techniques,* San Diego: Academic Press, Inc.; or Ausubel *et al.* (1987) *Current Protocols in Molecular Biology*, Greene Publishing and Wiley-Interscience, New York). Methods of screening human cDNA libraries for the *NBCCS* gene are provided in Example 1.

In another preferred embodiment, the human *PTC* cDNA can be isolated by amplification methods such as polymerase chain reaction (PCR). Table 2 provides primers suitable for the amplification of all 21 exons of the cDNA. In addition, appropriate PCR protocols are provided in Example 2.

### III. Expression of NBCCS polypeptides.

### A) De novo chemical synthesis.

The NBCCs proteins or subsequences thereof may be synthesized using standard chemical peptide synthesis techniques. Where the desired subsequences are relatively short (*e*.*g*., when a particular antigenic determinant is desired) the molecule may be synthesized as a single contiguous polypeptide. Where larger molecules are desired, subsequences can be synthesized separately (in one or more units) and then fused by condensation of the amino terminus of one molecule with the carboxyl terminus of the other molecule thereby forming a peptide bond.

Solid phase synthesis in which the C-terminal amino acid of the sequence is attached to an insoluble support followed by sequential addition of the remaining amino acids in the sequence is the preferred method for the chemical synthesis of the polypeptides of this invention. Techniques for solid phase synthesis are described by Barany and Merrifield, *Solid-Phase Peptide Synthesis;* pp. 3-284 in *The Peptides: Analysis, Synthesis, Biology. Vol. 2: Special Methods in Peptide Synthesis, Part A.,* Merrifield, *et al. J. Am. Chem. Soc.,* 85: 2149-2156 (1963), and Stewart *et al., Solid Phase Peptide Synthesis, 2nd ed.* Pierce Chem. Co., Rockford, III. (1984).

### B) Recombinant expression.

In a preferred embodiment, the NBCCS proteins or subsequences thereof, are synthesized using recombinant DNA methodology. Generally this involves creating a DNA sequence that encodes the fusion protein, placing the DNA in an expression cassette under the control of a particular promoter, expressing the protein in a host, isolating the expressed protein and, if required, renaturing the protein.

DNA encoding the NBCCS proteins or subsequences of this invention may be prepared by any suitable method as described above, including, for example, cloning and restriction of appropriate sequences or direct chemical synthesis by methods such as the phosphotriester method of Narang *et al. Meth. Enzymol*. 68 : 90-99 (1979); the phosphodiester method of Brown *et al., Meth. Enzymol*. 68: 109-151 (1979); the diethylphosphoramidite method of Beaucage *et al., Tetra. Lett*., 22: 1859-1862 (1981); and the solid support method of U.S. Patent No. 4,458,066.

Chemical synthesis produces a single stranded oligonucleotide. This may be converted into double stranded DNA by hybridization with a complementary sequence, or by polymerization with a DNA polymerase using the single strand as a template. One of skill would recognize that while chemical synthesis of DNA is limited to sequences of about 100 bases, longer sequences may be obtained by the ligation of shorter sequences.

Alternatively, subsequences may be cloned and the appropriate subsequences cleaved using appropriate restriction enzymes. The fragments may then be ligated to produce the desired DNA sequence.

In one embodiment, NBCCS proteins of this invention may be cloned using DNA amplification methods such as polymerase chain reaction (PCR). Thus, for example, the nucleic acid sequence or subsequence is PCR amplified, using a sense primer containing one restriction site (*e*.*g*., NdeI) and an antisense primer containing another restriction site (*e*.*g*., HindIII). This will produce a nucleic acid encoding the desired NBCCS sequence or subsequence and having terminal restriction sites. This nucleic acid can then be easily ligated into a vector containing a nucleic acid encoding the second molecule and having the appropriate corresponding restriction sites. Suitable PCR primers can be determined by one of skill in the art using the Sequence information provided in SEQ ID NO: 1. Appropriate restriction sites can also be added to the nucleic acid encoding the NBCCS protein or protein subsequence by site-directed mutagenesis. The plasmid containing the NBCCS sequence or subsequence is cleaved with the appropriate restriction endonuclease and then ligated into the vector encoding the second molecule according to standard methods.

The nucleic acid sequences encoding NBCCS proteins may be expressed in a variety of host cells, including *E. coli*, other bacterial hosts, yeast, and various higher eukaryotic cells such as the COS, CHO and HeLa cells lines and myeloma cell lines. As the NBCCS proteins are typically found in eukaryotes, a eukaryote host is preferred. The recombinant protein gene will be operably linked to appropriate expression control sequences for each host. For *E. coli* this includes a promoter such as the T7, trp, or lambda promoters, a ribosome binding site and preferably a transcription termination signal. For eukaryotic cells, the control sequences will include a promoter and preferably an enhancer derived from immunoglobulin genes, SV40, cytomegalovirus, etc., and a polyadenylation sequence, and may include splice donor and acceptor sequences.

The plasmids of the invention can be transferred into the chosen host cell by well-known methods such as calcium chloride transformation for *E. coli* and calcium phosphate treatment or electroporation for mammalian cells. Cells transformed by the plasmids can be selected by resistance to antibiotics conferred by genes contained on the plasmids, such as the amp, gpt, neo and hyg genes.

Once expressed, the recombinant NBCCS proteins can be purified according to standard procedures of the art, including ammonium sulfate precipitation, affinity columns, column chromatography, gel electrophoresis and the like (*see*, generally, R. Scopes, *Protein Purification,* Springer-Verlag, N.Y. (1982), Deutscher, *Methods in Enzymology Vol. 182: Guide to Protein Purification*., Academic Press, Inc. N.Y. (1990)). Substantially pure compositions of at least about 90 to 95% homogeneity are preferred, and 98 to 99% or more homogeneity are most preferred. Once purified, partially or to homogeneity as desired, the polypeptides may then be used (*e*.*g*., as immunogens for antibody production).

One of skill in the art would recognize that after chemical synthesis, biological expression, or purification, the NBCCS protein(s) may possess a conformation substantially different than the native conformations of the constituent polypeptides. In this case, it may be necessary to denature and reduce the polypeptide and then to cause the polypeptide to re-fold into the preferred conformation. Methods of reducing and denaturing proteins and inducing re-folding are well known to those of skill in the art (See, Debinski *et al*. (1993) *J. Biol. Chem.,* 268: 14065-14070; Kreitman and Pastan (1993) *Bioconjug. Chem.,* 4: 581-585; and Buchner, *et al*., (1992) *Anal. Biochem.,* 205: 263-270). Debinski *et al*., for example, describe the denaturation and reduction of inclusion body proteins in guanidine-DTE. The protein is then refolded in a redox buffer containing oxidized glutathione and L-arginine.

One of skill would recognize that modifications can be made to the NBCCS proteins without diminishing their biological activity. Some modifications may be made to facilitate the cloning, expression, or incorporation of the targeting molecule into a fusion protein. Such modifications are well known to those of skill in the art and include, for example, a methionine added at the amino terminus to provide an initiation site, or additional amino acids *(e.g.,* poly His) placed on either terminus to create conveniently located restriction sites or termination codons or purification sequences.

### IV. NBCCS (PTC) Therapeutics.

### A) Pharmaceutical Compositions

The NBCCS polypeptides of this invention are useful for parenteral, topical, oral, or local administration, such as by aerosol or transdermally, for prophylactic and/or therapeutic treatment. The pharmaceutical compositions can be administered in a variety of unit dosage forms depending upon the method of administration. For example, unit dosage forms suitable for oral administration include powder, tablets, pills, capsules and lozenges. It is recognized that the NBCCS polypeptides, when administered orally, must be protected from digestion. This is typically accomplished either by complexing the protein with a composition to render it resistant to acidic and enzymatic hydrolysis or by packaging the protein in an appropriately resistant carrier such as a liposome. Means of protecting proteins from digestion are well known in the art.

The pharmaceutical compositions of this invention are particularly useful for topical administration to treat basal cell carcinomas, or their precursors, solar keratoses. In another embodiment, the compositions are useful for parenteral administration, such as intravenous administration or administration into a body cavity or lumen of an organ. The compositions for administration will commonly comprise a solution of the NBCCS polypeptide dissolved in a pharmaceutically acceptable carrier, preferably an aqueous carrier. A variety of aqueous carriers can be used, *e*.*g*., buffered saline and the like. These solutions are sterile and generally free of undesirable matter. These compositions may be sterilized by conventional, well known sterilization techniques. The compositions may contain pharmaceutically acceptable auxiliary substances as required to approximate physiological conditions such as pH adjusting and buffering agents, toxicity adjusting agents and the like, for example, sodium acetate, sodium chloride, potassium chloride, calcium chloride, sodium lactate and the like. The concentration of chimeric molecule in these formulations can vary widely, and will be selected primarily based on fluid volumes, viscosities, body weight and the like in accordance with the particular mode of administration selected and the patient's needs.

Thus, a typical pharmaceutical composition for intravenous administration would be about 0.1 to 10 mg per patient per day. Dosages from 0.1 up to about 100 mg per patient per day may be used, particularly when the drug is administered to a secluded site and not into the blood stream, such as into a body cavity or into a lumen of an organ. Substantially higher dosages are possible in topical administration. Actual methods for preparing parenterally administrable compositions will be known or apparent to those skilled in the art and are described in more detail in such publications as *Remington's Pharmaceutical Science,* 15th ed., Mack Publishing Company, Easton, Pennsylvania (1980).

The compositions containing the present NBCCS polypeptides can be administered for therapeutic treatments. In therapeutic applications, compositions are administered to a patient suffering from a disease (*e.g.*, NBCCS or basal cell carcinoma) in an amount sufficient to cure or at least partially arrest the disease and its complications. An amount adequate to accomplish this is defined as a "therapeutically effective dose." Amounts effective for this use will depend upon the severity of the disease and the general state of the patient's health.

Single or multiple administrations of the compositions may be administered depending on the dosage and frequency as required and tolerated by the patient. In any event, the composition should provide a sufficient quantity of the proteins of this invention to effectively treat the patient.

Among various uses of the NBCCS polypeptides of the present invention are included a variety of disease conditions caused by nevoid basal cell carcinoma syndrome and/or basal cell carcinomas. Preferred applications include treatment of NBCCS, in particular treatment of the developmental anomalies characteristic of NBCCS and treatment of cancers, in particular basal cell carcinomas.

### B) Cellular Transformation and Gene Therapy.

The present invention provides packageable human *NBCCS* (*PTC*) nucleic acids (cDNAs) for the transformation of cells *in vitro* and *in vivo.* These packageable nucleic acids can be inserted into any of a number of well known vectors for the transfection and transformation of target cells and organisms as described below. The nucleic acids are transfected into cells, *ex vivo* or *in vivo,* through the interaction of the vector and the target cell. The NBCCS cDNA, under the control of a promoter, then expresses the NBCCS protein thereby mitigating the effects of absent NBCCS genes or partial inactivation of the NBCCS gene or abnormal expression of the NBCCS gene.

Such gene therapy procedures have been used to correct acquired and inherited genetic defects, cancer, and viral infection in a number of contexts. The ability to express artificial genes in humans facilitates the prevention and/or cure of many important human diseases, including many diseases which are not amenable to treatment by other therapies. As an example, *in vivo* expression of cholesterol-regulating genes, genes which selectively block the replication of HIV, and tumor-suppressing genes in human patients dramatically improves the treatment of heart disease, AIDS, and cancer, respectively. For a review of gene therapy procedures, see Anderson, *Science* (1992) 256:808-813; Nabel and Felgner (1993) *TIBTECH* 11: 211-217; Mitani and Caskey (1993) *TIBTECH* 11: 162-166; Mulligan (1993) *Science* 926-932; Dillon (1993) *TIBTECH* 11: 167-175; Miller (1992) *Nature* 357: 455-460; Van Brunt (1988) *Biotechnology* 6(10): 1149-1154; Vigne (1995) *Restorative Neurology and Neuroscience* 8: 35-36; Kremer and Perricaudet (1995) *British Medical Bulletin* 51(1) 31-44; Haddada *et al.* (1995) in *Current Topics in Microbiology and Immunology* Doerfler and Böhm (eds) Springer-Verlag, Heidelberg Germany; and Yu *et al., Gene Therapy* (1994) 1:13-26.

Delivery of the gene or genetic material into the cell is the first critical step in gene therapy treatment of disease. A large number of delivery methods are well known to those of skill in the art. Such methods include, for example liposome-based gene delivery (Debs and Zhu (1993) WO 93/24640; Mannino and Gould-Fogerite (1988) *BioTechniques* 6(7): 682-691; Rose U.S. Pat No. 5,279,833; Brigham (1991) WO 91/06309; and Felgner *et al.* (1987) *Proc. Natl. Acad. Sci. USA* 84: 7413-7414), and replication-defective retroviral vectors harboring a therapeutic polynucleotide sequence as part of the retroviral genome (*see, e.g.,* Miller *et al.* (1990) *Mol. Cell. Biol*. 10:4239 (1990); Kolberg (1992) *J. NIH Res.* 4:43, and Cometta et al. *Hum. Gene Ther.* 2:215 (1991)). Widely used retroviral vectors include those based upon murine leukemia virus (MuLV), gibbon ape leukemia virus (GaLV), Simian Immuno deficiency virus (SIV), human immuno deficiency virus (HIV), and combinations thereof. *See, e.g.,* Buchscher *et al.* (1992) *J. Virol*. 66(5) 2731-2739; Johann *et al.* (1992) *J. Virol.* 66 (5): 1635-1640 (1992); Sommerfelt *et al.,* (1990) *Virol.* 176:58-59; Wilson *et al.* (1989) *J. Virol.* 63:2374-2378; Miller et al., *J. Virol.* 65:2220-2224 (1991); Wong-Staal *et al*., PCT/US94/05700, and Rosenburg and Fauci (1993) in *Fundamental Immunology, Third Edition* Paul (ed) Raven Press, Ltd., New York and the references therein, and Yu *et al., Gene Therapy* (1994) *supra*).

AAV-based vectors are also used to transduce cells with target nucleic acids, *e.g.,* in the *in vitro* production of nucleic acids and peptides, and in *in vivo* and *ex vivo* gene therapy procedures. *See,* West *et al*. (1987) *Virology* 160:38-47; Carter *et al.* (1989) U.S. Patent No. 4,797,368; Carter *et al.* WO 93/24641 (1993); Kotin (1994) *Human Gene Therapy* 5:793-801; Muzyczka (1994) *J. Clin. Invest.* 94:1351 and Samulski (*supra*) for an overview of AAV vectors. Construction of recombinant AAV vectors are described in a number of publications, including Lebkowski, U.S. Pat. No. 5,173,414; Tratschin *et al.* (1985) *Mol. Cell. Biol*. 5(11):3251-3260; Tratschin, *et al.* (1984) *Mol. Cell. Biol.* 4:2072-2081; Hermonat and Muzyczka (1984) *Proc. Natl. Acad. Sci. USA* 81:6466-6470; McLaughlin *et al.* (1988) and Samulski *et al.* (1989) *J. Virol.* 63:03822-3828. Cell lines that can be transformed by rAAV include those described in Lebkowski *et al.* (1988) *Mol. Cell. Biol.* 8: 3988-3996.

### A) Ex vivo transformation of cells.

*Ex vivo* cell transformation for diagnostics, research, or for gene therapy (*e.g.,* via re-infusion of the transformed cells into the host organism) is well known to those of skill in the art. In a preferred embodiment, cells are isolated from the subject organism, transfected with the NBCCS gene or cDNA of this invention, and re-infused back into the subject organism (*e.g.*, patient). Various cell types suitable for *ex vivo* transformation are well known to those of skill in the art. Particular preferred cells are progenitor or stem cells (*see, e.g.,* Freshney *et al., Culture of Animal Cells, a Manual of Basic Technique, third edition* Wiley-Liss, New York (1994)) and the references cited therein for a discussion of how to isolate and culture cells from patients).

As indicated above, in a preferred embodiment, the packageable nucleic acid encodes an *NBCCS* polypeptide under the control of an activated or constitutive promoter. The transformed cell(s) express functional *NBCCS* polypeptide which mitigates the effects of deficient or abnormal NBCCS gene expression.

In one particularly preferred embodiment, stem cells are used in *ex-vivo* procedures for cell transformation and gene therapy. The advantage to using stem cells is that they can be differentiated into other cell types *in vitro,* or can be introduced into a mammal (such as the donor of the cells) where they will engraft in the bone marrow. Methods for differentiating CD34⁺ cells *in vitro* into clinically important immune cell types using cytokines such a GM-CSF, IFN-γ and TNF-α are known *(see,* Inaba *et al.* (1992) *J. Exp. Med* 176: 1693-1702, and Szabolcs *et al*. (1995) 154: 5851-5861).

Stem cells are isolated for transduction and differentiation using known methods. For example, in mice, bone marrow cells are isolated by sacrificing the mouse and cutting the leg bones with a pair of scissors. Stem cells are isolated from bone marrow cells by panning the bone marrow cells with antibodies which bind unwanted cells, such as CD4⁺ and CD8⁺ (T cells), CD45⁺ (panB cells), GR-1 (granulocytes), and Ia^{d} (differentiated antigen presenting cells). *For an example of this protocol see,* Inaba *et al.* (1992) *J. Exp. Med* 176: 1693-1702.

In humans, bone marrow aspirations from iliac crests are performed *e.g.*, under general anesthesia in the operating room. The bone marrow aspirations are approximately 1,000 ml in quantity and are collected from the posterior iliac bones and crests. If the total number of cells collected is less than about 2 x 10⁸/kg, a second aspiration using the sternum and anterior iliac crests in addition to posterior crests is performed. During the operation, two units of irradiated packed red cells are administered to replace the volume of marrow taken by the aspiration. Human hematopoietic progenitor and stem cells are characterized by the presence of a CD34 surface membrane antigen. This antigen is used for purification, *e.g.,* on affinity columns which bind CD34. After the bone marrow is harvested, the mononuclear cells are separated from the other components by means of Ficoll gradient centrifugation. This is performed by a semi-automated method using a cell separator (*e.g.*, a Baxter Fenwal CS3000+ or Terumo machine). The light density cells, composed mostly of mononuclear cells are collected and the cells are incubated in plastic flasks at 37°C for 1.5 hours. The adherent cells (monocytes, macrophages and B-Cells) are discarded. The non-adherent cells are then collected and incubated with a monoclonal anti-CD34 antibody (*e*.*g*., the murine antibody 9C5) at 4°C for 30 minutes with gentle rotation. The final concentration for the anti-CD34 antibody is 10 µg/ml. After two washes, paramagnetic microspheres (DynaBeads, supplied by Baxter Immunotherapy Group, Santa Ana, California) coated with sheep antimouse IgG (Fc) antibody are added to the cell suspension at a ratio of 2 cells/bead. After a further incubation period of 30 minutes at 4°C, the rosetted cells with magnetic beads are collected with a magnet. Chymopapain (supplied by Baxter Immunotherapy Group, Santa Ana, California) at a final concentration of 200 U/ml is added to release the beads from the CD34+ cells. Alternatively, and preferably, an affinity column isolation procedure can be used which binds to CD34, or to antibodies bound to CD34 (*see,* the examples below). *See,* Ho *et al*. (1995) *Stem Cells* 13 (suppl. 3): 100-105. *See also,* Brenner (1993) *Journal of Hematotherapy* 2: 7-17.

In another embodiment, hematopoietic stem cells are isolated from fetal cord blood. Yu *et al*. (1995) *Proc. Natl. Acad. Sci. USA* 92: 699-703 describe a preferred method of transducing CD34⁺ cells from human fetal cord blood using retroviral vectors.

### B) In vivo transformation

Vectors (*e.g.*, retroviruses, adenoviruses, liposomes, *etc.*) containing therapeutic nucleic acids can be administered directly to the organism for transduction of cells *in vivo.* Administration is by any of the routes normally used for introducing a molecule into ultimate contact with blood or tissue cells. The packaged nucleic acids are administered in any suitable manner, preferably with pharmaceutically acceptable carriers. Suitable methods of administering such packaged nucleic acids are available and well known to those of skill in the art, and, although more than one route can be used to administer a particular composition, a particular route can often provide a more immediate and more effective reaction than another route.

Pharmaceutically acceptable carriers are determined in part by the particular composition being administered, as well as by the particular method used to administer the composition. Accordingly, there is a wide variety of suitable formulations of pharmaceutical compositions of the present invention.

Formulations suitable for oral administration can consist of (a) liquid solutions, such as an effective amount of the packaged nucleic acid suspended in diluents, such as water, saline or PEG 400; (b) capsules, sachets or tablets, each containing a predetermined amount of the active ingredient, as liquids, solids, granules or gelatin; (c) suspensions in an appropriate liquid; and (d) suitable emulsions. Tablet forms can include one or more of lactose, sucrose, mannitol, sorbitol, calcium phosphates, corn starch, potato starch, tragacanth, microcrystalline cellulose, acacia, gelatin, colloidal silicon dioxide, croscarmellose sodium, talc, magnesium stearate, stearic acid, and other excipients, colorants, fillers, binders, diluents, buffering agents, moistening agents, preservatives, flavoring agents, dyes, disintegrating agents, and pharmaceutically compatible carriers. Lozenge forms can comprise the active ingredient in a flavor, usually sucrose and acacia or tragacanth, as well as pastilles comprising the active ingredient in an inert base, such as gelatin and glycerin or sucrose and acacia emulsions, gels, and the like containing, in addition to the active ingredient, carriers known in the art.

The packaged nucleic acids, alone or in combination with other suitable components, can be made into aerosol formulations (*i.e.*, they can be "nebulized") to be administered via inhalation. Aerosol formulations can be placed into pressurized acceptable propellants, such as dichlorodifluoromethane, propane, nitrogen, and the like.

Suitable formulations for rectal administration include, for example, suppositories, which consist of the packaged nucleic acid with a suppository base. Suitable suppository bases include natural or synthetic triglycerides or paraffin hydrocarbons. In addition, it is also possible to use gelatin rectal capsules which consist of a combination of the packaged nucleic acid with a base, including, for example, liquid triglycerides, polyethylene glycols, and paraffin hydrocarbons.

Formulations suitable for parenteral administration, such as, for example, by intraarticular (in the joints), intravenous, intramuscular, intradermal, intraperitoneal, and subcutaneous routes, include aqueous and non-aqueous, isotonic sterile injection solutions, which can contain antioxidants, buffers, bacteriostats, and solutes that render the formulation isotonic with the blood of the intended recipient, and aqueous and non-aqueous sterile suspensions that can include suspending agents, solubilizers, thickening agents, stabilizers, and preservatives. In the practice of this invention, compositions can be administered, for example, by intravenous infusion, orally, topically, intraperitoneally, intravesically or intrathecally. Parenteral administration and intravenous administration are the preferred methods of administration. The formulations of packaged nucleic acid can be presented in unit-dose or multi-dose sealed containers, such as ampules and vials.

Injection solutions and suspensions can be prepared from sterile powders, granules, and tablets of the kind previously described. Cells transduced by the packaged nucleic acid as described above in the context of *ex vivo* therapy can also be administered intravenously or parenterally as described above.

The dose administered to a patient, in the context of the present invention should be sufficient to effect a beneficial therapeutic response in the patient over time. The dose will be determined by the efficacy of the particular vector employed and the condition of the patient, as well as the body weight or surface area of the patient to be treated. The size of the dose also will be determined by the existence, nature, and extent of any adverse side-effects that accompany the administration of a particular vector, or transduced cell type in a particular patient.

In determining the effective amount of the vector to be administered in the treatment or prophylaxis NBCCS predilection or onset or basal cell carcinoma predilection or onset, the physician evaluates circulating plasma levels of the vector, vector toxicities, progression of the disease, and the production of anti-vector antibodies. In general, the dose equivalent of a naked nucleic acid from a vector is from about 1 µg to 100 µg for a typical 70 kilogram patient, and doses of vectors which include a retroviral particle are calculated to yield an equivalent amount of therapeutic nucleic acid.

For administration, inhibitors and transduced cells of the present invention can be administered at a rate determined by the LD-50 of the inhibitor, vector, or transduced cell type, and the side-effects of the inhibitor, vector or cell type at various concentrations, as applied to the mass and overall health of the patient. Administration can be accomplished via single or divided doses.

In a preferred embodiment, prior to infusion, blood samples are obtained and saved for analysis. Between 1 X 10⁸ and 1 X 10¹² transduced cells are infused intravenously over 60- 200 minutes. Vital signs and oxygen saturation by pulse oximetry are closely monitored. Blood samples are obtained 5 minutes and 1 hour following infusion and saved for subsequent analysis. Leukopheresis, transduction and reinfusion can be repeated are repeated every 2 to 3 months. After the first treatment, infusions can be performed on a outpatient basis at the discretion of the clinician. If the reinfusion is given as an outpatient, the participant is monitored for at least 4, and preferably 8 hours following the therapy.

Transduced cells are prepared for reinfusion according to established methods. See, Abrahamsen *et al*. (1991) *J. Clin. Apheresis,* 6: 48-53; Carter *et al*. (1988) *J. Clin. Apheresis*, 4:113-117; Aebersold *et al*. (1988) *J. Immunol. Meth.,* 112: 1-7; Muul *et al*. (1987) *J. Immunol. Methods* 101:171-181 and Carter *et al.* (1987) *Transfusion* 27: 362-365. After a period of about 2-4 weeks in culture, the cells should number between 1 x 10⁸ and 1 x 10¹². In this regard, the growth characteristics of cells vary from patient to patient and from cell type to cell type. About 72 hours prior to reinfusion of the transduced cells, an aliquot is taken for analysis of phenotype, and percentage of cells expressing the therapeutic agent.

### EXAMPLES

The following examples are offered to illustrate, but not to limit the present invention.

### Example 1

### Cloning of a Human Patched Homologue

This example describes the isolation of a complete human *PATCHED* cDNA sequence which encodes a putative protein of 1172 amino acids, and displays 61 % sequence identity to the Drosophila *PATCHED* protein. *Drosophila patched (ptc)* is a segment polarity gene required for the correct patterning of larval segments and imaginal discs during fly development (Nakano *et al.* (1989) *Nature* 341: 508-13; Hooper *et al.* (1989) *Cell* 59: 751-765). Based on genetic studies, patched is a component of the signaling pathway of the morphogen *hedgehog* (Basler *et al.* (1994) *Nature* 368: 208-214; Capdevila *et al.* (1994) *EMBO J*. 13: 71-82; Ingham (1991) *Nature* 353: 184-187). Since patched is a putative membrane-spanning protein, and is expressed in *hedgehog* responsive cells, it has been proposed to be the *hedgehog* receptor (Ingham (1991) *supra*.).

In vertebrates, several hedgehog homologs have been identified. The best characterized of them, *sonic hedgehog,* has been implicated in the dorsal-ventral patterning of neural tube (Roelink *et al.* (1994) *Cell* 76: 761-775; Roelink (1995) *Cell* 81: 445-455), in the differentiation of somites (Johnson *et al.* (1994) *Cell* 79: 1165-1173) and in the establishing of the anterior-posterior axis of the limb bud (Riddle *et al.* (1993) *Cell,* 75: 1401-1416). The biochemical basis of hedgehog signaling in vertebrates remains poorly understood and has been hampered largely by the lack of a proven receptor for the molecule.

### Experimental Procedures

### Cosmid Isolation

Cosmids used in this study were isolated from a human chromosome 9-specific genomic cosmid library (LL09NCO1"P", Biomedical Sciences Division, Lawrence Livermore National Laboratory, Livermore, CA 94550) by screening with the YAC clone ICI-2ef8 (UK Human Genome Mapping Project Resource Centre). This clone contains the microsatellite marker *D9S287* which has been localized to chromosome 9q22.3 (Povey *et al.* (1994) *Ann. Hum. Genet.,* 58: 177-250). The isolation of YAC DNA an hybridization was performed as described by Vorechovsky *et al*. (1994) *Genomics*, 21: 517-24. The localization of the cosmids was confirmed by hybridization to YAC ICI-2ef8 resolved by means of pulse-field gel electrophoresis. The 96 well plate format of the cosmid clones that contain PTC is 42HI1, 96F9, 218A8, 226G7.

### Library screening

Human cDNA clones were isolated from a fetal brain cDNA library in the lambda ZAPII phase vector (Stratagene, La Jolla, California, USA), using standard procedures. The probes were labeled with [³²P]dCTP by random priming (Redisrime^{™}, Amersham). Positive clones were rescued using the 704 helper phase/pBluescript^{™} excision system (Rapid Excision Kit^{™}, Stratagene) and sequenced. Mouse genomic clones were isolated from a 129SV lambda Fix II^{™} library (Stratagene). Phase DNA was cut with *Eco*RI and hybridized with *PTC* specific probes. Mouse cDNA clones were isolated from an 11.5 dpc mouse embryo (Swiss male) library constructed in lambda gt10. Hybridization was performed at 55°C. Positive clones were subcloned into pBluescript^{™}II SK (Stratagene) digested with *Not*I.

### Sequencing

Templates for sequencing were prepared from overnight cultures of rescued cDNA clones and/or *Eco*RI cosmid fragments subcloned in pBluescript^{™}KS(+) using a plasmid purification kit (Qiagen). Sequencing was performed with the Taq Dyedeoxy^{™} Terminator Cycle sequencing kit (Applied Biosystems) according to the manufacturer's instructions. Sequencing reactions were resolved on an ABI 373A automated sequencer. Sequence analysis was performed using the GCG software. BLAST searches were performed with the NCBI network service. PTC sequences have been deposited in GENBANK under accession #U43148.

### Northern hybridization

Expression of human PTC mRNA was examined by Northern hybridization of human tissue blots (Clontech) using cDNA probes labeled with [³²P]dCTP. Hybridization solution contained 5x SSPE, 10x Denhardt's solution, 100 mg/ml denatured, sheared herring sperm DNA, 50% formamide and 2% SDS. Washes were performed at 60°C with 2x SSC and 0.1% SDS.

### Chromosomal localization

The chromosomal localization of human *PTC* was identified by PCR analysis of DNA panels obtained from human-hamster hybrid cells. The panel consisted of both whole chromosome 9 hybrids and deletion hybrids of 9q22.3. The primers used were PTC1 (5'-TTG CAT AAC CAG CGA GTCT-3' (SEQ ID NO: 2)) and PTC2 (5'-CAA ATG TAC GAG CAC TTC AAGG-3' (SEQ ID NO: 3)). Murine *Ptc* was mapped by means of interspecific backcross mapping. The panels were provided by the Jackson Laboratory (Bar Harbor, ME) and are the BSB panel from a cross (C57BL/6J x *M. spretus*) x C57BL/6J and a similar BSS panel made up of DNA from the reciprocal backcross (C57BL/6JEi x SPRET/Ei) x SPRET/Ei. Mapping was performed by means of SSCP (single strand conformation polymorphism) analysis with the primers W18F3 (5'-CTG TCA AGG TGA ATG GAC-3' (SEQ ID NO: 4) and W18R3 (5'-GGG GTT ATT CTG TAA AAGG-3' (SEQ ID NO: 5)). PCR reactions were performed in the presence of [³²P]dCTP. The samples were resolved on a 6% acrylamide gel (2.6% cross-linking) at 4°C at 70 watts within 1.5 hours. Genetic linkage was performed by segregation analysis.

### In situ hybridization

Whole mount *in situ* hybridization on mouse embryos and subsequent sectioning was performed as described by Christiansen, *et al.* (1995) *Mech. Dev.* 51: 341-50. The mouse *Ptc* probe was a 706 bp *Not*1/*Pst*I cDNA fragment from the 5' end of the gene, subcloned in pBluescriptII SK. The probe was linearized with *Sac*II, the overhang blunted by incubation with 5 U/mg Klenow at 22°C for 15 minutes, and antisense RNA synthesized by transcribing with T7 RNA polymerase.

### Results and Discussion

### Cloning of a human PTC homolog

Cosmids used in this study were isolated from a human chromosome 9-specific genomic cosmid library using the YAC clone ICI-2ef8. This clone contains the microsatellite marker D9S287 which has been localized to chromosome 9q22.3. Sequencing of a 1.8 kb *Eco*RI fragment of cosmid 42Hll yielded an open reading frame with significant homology to three consecutive stretches of the *Drosophila ptc* protein. Using the 1.8 kb *Eco*RI fragment as a probe the complete human and partial mouse *PTC* cDNA sequences were isolated.

The sequence of the human *PTC* cDNA consists of an open reading frame of 3888 nucleotides; also sequenced were 441 and 2240 nucleotides on the 5' end and on the 3' end, respectively (SEQ ID NO: 1; Figure 8). The open reading frame of human *PTC* cDNA encodes for a putative protein of 1296 amino acids. This open reading frame is initiated by an ATG codon that has a moderate match for the translational start consensus sequence in vertebrates (GAGGCTAUGT (SEQ ID NO: 6) in *PTC* versus GCCGCCAUGG (SEQ ID NO: 7) (Kozak (1991) *J. Biol. Chem.,* 266: 19867-19870)). Assuming that this codon encodes for the first amino acid of the protein, human *ptc* consists of 1296 amino acids with a relative molecular weight (Mᵣ) of 131 x 10³. It shows 61 % sequence identity to its *Drosophila* counterpart. Upstream of the ATG, the open reading frame extends for another 354 nucleotides (starting at base pair 88 of the sequence shown in Figure 8). The 3' untranslated region contains a canonical polyadenylation signal (AATAAA (SEQ ID NO: 8)) as well as mRNA destabilizing ATTTA (SEQ ID NO: 9) motifs.

An alternative transcript is observed that splices from exon 3 to exon 2a. The open reading frame ends in exon 2a (*see,* SEQ ID NO: 59) but does not contain an AUG. Exon 2 can splice to one of three different first exons. Exon 1b (*see* SEQ ID NO: 58) is homologous to the described first exon of the mouse mRNA and has an ATG followed by ORF. Exon 1a has ORF through the entire length and a potential splice acceptor site (*see, e.g.,* SEQ ID NO: 58). Exon 3 contains the first in-frame ATG for all the transcripts except the one initiating in exon 1b. A map of the promoter region of *NBCCS* (*PCT*) is provided in Figure 3.

Hydropathy analysis (Kyte *et al*. (1982) *J. Mol. Biol*., 157: 105-32) of the entire open reading frame of human *PTC* predicts the presence of eight main hydrophobic stretches. Distribution of the hydrophobic blocks is remarkably well conserved between species indicating that human *PTC,* like its Drosophila counterpart, is an integral membrane protein.

### Chromosomal localization of PTC

Chromosomal localization of human *PTC* on 9q22.3 was confirmed by PCR analysis of chromosome 9 hybrids, and deletion hybrids of 9q22.3, human-hamster hybrid DNA panels. The primers used (PTC1, PTC2) were derived from a sequence of a 1.8 kb *Eco*RI fragment of cosmid 42Hll. Primer PTC1 is derived from an exon sequence and PTC2 from an intron sequence. All DNA hybridization and cDNA sequencing data suggest that human PTC is a single copy gene. Murine *Ptc* maps to a short region of chromosome 13, close to the murine Facc locus (no recombination out of 188 meioses). This region contains the mouse mutations *flexed tail* (*f*) and purkinje cell degeneration (*pcd*), and it is syntenic with human 9q22-q31. Both *f* and *pcd* involve abnormal development of cells of the bone or brain and could be allelic to *Ptc*.

### Expression of PTC

Northern blot analysis revealed five distinct *PTC* transcripts in all human tissues examined. Expression of these transcripts appears to be differentially regulated. During mouse embryogenesis, expression of *Ptc* is first detected at E 8.0 dpc in ventral neuroepithelial tissue in two separate domains along the midline. Expression persists in ventral neural cells through to 9.5 dpc and transcripts are also detected in lateral mesenchyme surrounding the neural tube. *Ptc* transcription is detected in the somites soon after the time of their appearance and follows a rostro-caudal gradient of expression. Somite expression is restricted to epithelial cells within the medial aspects of each somite. Expression of *Ptc* is also detected in the posterior ectoderm of each limb bud from 10.0 dpc to 12. 5 dpc. This region corresponds to surface ectoderm that covers the ZPA. Other sites of *Ptc* expression during this period include the inner surfaces of the branchial arches which flank the oropharyngeal region, cells surrounding the placodes of the vibrissae and the genital eminence.

The expression pattern of *Ptc* points to a close relationship between *Ptc* and the hedgehog family of morphogens. This relationship was originally established in *Drosophila* (Ingham *et al*. (1991) *Nature,* 353: 184-187). In vertebrates, the best characterized *hedgehog* homolog, *sonic hedgehog,* has been implied in the induction of the floorplate and motor neurons within the ventral neural tube (Jessell *et al.* (1990) *Harvey* Lect., 86: 87-128; Yamada *et al*. (1993), 73: 673-686) as well as in the differentiation of sclerotome within the somites (Pourquie *et al.* (1993) *Proc. Natl. Acad Sci. USA,* 90: 5242-5246). In the limb bud, *sonic hedgehog* expression in the mesenchymal 'zone of polarizing activity' triggers anterior-posterior patterning of the limb (Riddle *et al*. (1993) *Cell,* 75: 1401-1416). Our data show that vertebrate PTC is expressed in all major target tissues of *sonic hedgehog,* such as the ventral neural tube, somites and tissues surrounding the zone of polarizing activity of the limb bud. The striking spatial complementarity and temporal coincidence of the *sonic hedgehog* and *Ptc* expression patterns suggest that both genes might be members of a common signaling pathway.

The localization of *PTC* in the region containing the nevoid basal cell carcinoma syndrome (NBCCS) gene is intriguing. NBCCS is an autosomal dominant disorder which predisposes affected individuals to basal cell carcinomas of the skin, medulloblastomas and various other tumors (Gorlin (1987) *Medicine (Baltimore)* 66: 98-113). Recent genetic studies have placed the gene for the nevoid basal cell carcinoma syndrome to chromosome 9q22.3, between the markers Fanconi anaemia complementation group A (Farndon *et al.* (1994) *Genomics,* 23: 486-489) and *D9S287* (Periçak-Vance (1995) *Ann. Hum. Genet.,* 59: 347-365). Several lines of evidence suggest that *PTC* is a candidate gene for the nevoid basal cell carcinoma syndrome. *Ptc* expression is compatible with the congenital defects commonly found in NBCCS patients. Frequent symptoms in newborns and infants are developmental anomalies of the spine and ribs (Gorlin (1987 *supra*.). These malformations could be due to a *PTC* deficiency, expression of which coincides spatially and temporally with the development of the neural tube and of the somites. In addition, *Ptc* expression in the surface ectoderm surrounding the ZPA is consistent with limb abnormalities often observed in the patients with NBCCS (Gorlin (1987 *supra.*). *PTC* expression in all adult tissues points to a pleiotropic role of *PTC* in adult signal transduction pathways. Defects in these signaling pathways could account for the symptoms which develop postnatally.

### Example 2

### Mutations of the Human Homologue of Drosophila Patched in Nevoid Basal Cell Carcinoma Syndrome

The nevoid basal cell carcinoma syndrome (NBCCS) is an autosomal dominant disorder characterized by multiple basal cell carcinomas (BCCs), pits of the palms and soles, keratocysts of the jaw, and a variety of other tumors and developmental abnormalities. NBCCS was mapped to chromosome 9q22.3 and both familial and sporadic BCCs display loss of heterozygosity for markers in this region, consistent with the gene being a tumor suppressor. Example I describes the isolation of a human sequence (*PTC*) with strong homology to the *Drosophila* segment polarity gene. This example shows that human *PTC* is expressed in many of the tissues affected in NBCCS patients. Single-stranded conformation polymorphism analysis and sequencing revealed mutations of *PTC* in patients with the syndrome and in related tumors. The data indicate that human *PTC* is also an *NBCCS* gene and that a reduction in expression of this gene leads to the developmental abnormalities observed in the syndrome and that complete loss of patched function contributes to transformation of certain cell types.

### Experimental Procedures

### Subjects and samples

DNA samples were collected from 363 individuals in 128 NBCCS kindreds. Patients were examined by a clinical geneticist, and diagnosis of Gorlin syndrome was based on at least two major features of the syndrome; *e.g.*, jaw cysts, palmar pits, multiple basal cell carcinomas, and a family history of typical Gorlin syndrome. Lymphoblastoid cell lines were made from at least one affected member of 82 kindreds. 252 basal cell carcinomas were collected as either fresh or paraffin-embedded specimens.

### Short tandem repeat polymorphisms

For linkage analysis and tumor deletion studies, PCR reactions were performed in 50 µL volumes containing 100 ng of template DNA, 200 M dNTPs, 1.5 MM MgCl₂, 0.25 mM spermidine, 10 pM of each primer, I Ci ³²P dCTP (Amersham, Arlington Heights, Illinois, USA), and 1.25 Units Taq polymerase (Promega, Madison, Wisconsin, USA) in Promega buffer (10 mM TrisHCl, pH 9, 50 mM KCl, 0.1% Triton X-100^{™}). An Ericomp Dual Block^{™} thermocyler was set with the following parameters for 25 cycles: 94°C, 1 mm, 55°C, 30 sec, 72°C, 2 min. PCR products were analyzed on an 5% polyacrylamide gels. Autoradiography was carried out at -70°C with Kodak XAR^{™} film. Loci in the *NBCCS* region that were typed are shown in Figure 1, and primer sequences are available from the Genome Data Base (http://gdbwww.gdb.org).

### Pulsed-field gel electrophoresis (PFGE)

Cultured lymphoblastoid cells were embedded in LMP agarose (Bio Rad, Hercules California, USA) at a concentration of approximately 2 x 10⁶/220- 1 block, and DNA was extracted according to standard methods (Sambrook *et al. supra.*) Quarter blocks were digested with SacII, MluI, NotI, BssHI, NruI, and SfiI under conditions recommended by the manufacturer (New England Biolabs, Beverly, Massachusetts, USA). Electrophoresis was carried out with the Bio Rad CHEF DR 11 apparatus using, 1% agarose gels run for 20 hours at 200 volts with a pulse time of 75 sec. For higher resolution of fragments under 500 kb, a 25 second pulse time was used.

Transfer to nylon membranes (Du Pont Gene Screen Plus^{™}, Du Pont, Co., Boston, Massachusetts, USA) was performed according to the manufacturer's instruction after exposure of the gel to UV (6-7 mW/cm²) for two minutes. Probes were labeled to a specific activity of approximately 10⁹ DPM/g, with dCT³²P (Amersham) by the random primed synthesis method (Boehringer Mannheim Kit, Boehringer Mannheim Corp., Indianapolis, Indiana, USA). Hybridization was carried out for 18 hours at 65°C in 0.5 M sodium phosphate (pH 7.2), 7% SDS, 1% BSA, 1 mM EDTA and 200 µg/ml herring sperm. For probes containing repetitive sequences, sheared, sonicated human placental DNA (Sigma Chemical Co., St. Louis, Missouri, USA) was added to the hybridization solution (500 µg/ml) and preassociated with the probe at 65°C for 45 minutes prior to hybridization to the filter. Filters were washed in 0.1 X SSC with 1% SDS at 65°C and exposed to autoradiographic film with an intensifying screen at -70°C from 12 hours to three days. Probes that detected similar sized fragments on different blots were directly compared for comigrating fragments by comigrating fragments by hybridization to the same blot. Blots were stripped in 0.4N NaOH for 30 min at room temperature between uses.

### Fluorescence in situ hybridization

Cosmid clones were labeled by nick translation with biotin-11-dUTP, dioxigenin-11-dUTP, or both, and hybridized to metaphase and interphase chromosomes under suppression conditions. Biotinylated probes were detected with 5 µg/ml of fluorescein isothiocyanate (FITC)-conjugated avidin DCS. Dioxigenin labeled probes were detected with 2 µg/ml anti-dioxigenin Fab conjugated to rhodamine. The chromosomes were counterstained with 200 ng/ml of 4,6-diamidino-2-phenylindole-dihydrochloride (DAPI). Images were obtained using, a microscope coupled to a cooled CCD camera. The digitalized images were processed, pseudocolored and merged and the distances between signals were measured.

### Cosmid and BAC screening

A gridded chromosome 9 cosmid library (LL09NCO1) was replicated onto nylon filters (Gene Screen Dupont Plus, Du Pont Co.) and screened according to the recommendations of the Human Genome Center, Lawrence Livermore National Laboratory. Positive coordinates were streaked out to single colonies and confirmed to contain the appropriate markers by PCR or hybridization. Gridded BAC filters were screened by hybridization according to the manufacturer's recommendations (Research Genetics, Huntsville, Alabama, USA). Because of the small chance of chimerism in cosmids and BACS, fragments from the ends of contigs were mapped with a panel of human-hamster somatic cell hybrids to confirm their localization on chromosome 9q22.

### Isolation of cDNAs

Four methods were used to isolate candidate cDNAs. Direct cDNA selection (Parimoo *et al.* (1991) *Proc. Natl. Acad. Sci., USA,* **88:** 9623-9627) was applied to pools of cosmids and BACs. Following two rounds of selection, the PCR products were size fractionated and cloned into PCRII (Invitrogen, Leek NV, Netherlands). Transformants were gridded into 96 well plates, and replica filters were probed with the genomic template DNA to identify cDNAs that hybridized the correct genomic region.

Exon trapping, was performed using the method developed by Buckler *et al*. (1991) *Proc. Natl. Acad Sci. USA*, 88: 4005-4009, and later modified by Church *et al*. (1994) *Nature Genetics,* 6: 98. BamHl/GblII digests of pools of 5 or 6 cosmids were cloned into the BamHl site of the splicing vector pSPL3b (Burn *et al.* (1995) *Gene,* 161: 183-187). Trapped DNAs were sequenced and mapped back to the NBCCS candidate region by hybridization to the cosmids from which they were derived.

For HTF island cloning, YACs were size fractionated by pulsed field gel electrophoresis, excised from the gel, and digested with BssHII. Subsequently vectorette linkers were added and PCR amplification was performed using a vectorette primer and a 5' Alu primer (Valdes *et al*. (1994) *Proc. Natl. Acad. Sci.,* 91: 5377-5381). After an initial denaturation at 100°C for 5 min, 30 amplification cycles were performed with denaturation for 1 min at 98°C, annealing for 1 min at 60°C, and extension for 3 min at 72°C. Ten units of Taq polymerase were used in a total volume of 100 µl consisting of 50 mM KCl, 10 mM Tris pH 9, 2 mM MgCl₂, 0.1 % Triton and 200 µM dNTP. The PCR products were electrophoresed on a 1% agarose gel, in order to determine their size, and subsequently cloned into the PGEM T vector (Promega) by a shotgun procedure.

For sequence sampling, the ends of chromosome 9 specific cosmids or cosmid subclones were directly sequenced (Smith *et al*. (1994) *Nature Genetics* 7: 40-47. Sequencing was performed on an ABI 373 DNA sequencer. The resulting, end sequences were manually trimmed, examined for simple sequence repeats, and used to search the DNA sequence databases. Both nucleotide and amino acid searches were performed. In addition sequences were examined for potential coding regions by GRAIL (Uberbacher and Mural *(1991) Proc. Natl. Acad. Sci. USA* 88: 11261-11265.

Short cDNA fragments obtained by the methods outlined above were extended by screening brain or epidermal cDNA libraries and by rapid amplification of cDNA ends (Marathon kit, Clontech, Palo Alto, California, USA).

### Intron/exon structure of the human patched gene

Oligonucleotides were chosen at approximately 150 bp intervals spanning the cDNA of the human patched gene. PCR products were generated from cosmids 226G7, 42H11, 55A16, or 96F9. Reactions were performed in a 50 µl volume containing 25 pmol of various oligonucleotide combinations, 200 µmol dNTPs, 1.5 mM, or 1.85 mM, or 2.2 mM MgCl₂, 5 U Taq polymerase, and amplified for 35 cycles of 94°C for 30 s, 55°C for 30 s and 72° C for 2.5 min. Some samples were amplified by long range PCR using the Expand Long Template PCR system (Boehringer Mannheim) according to the manufacturer's instructions. PCR products were resolved on a 1% agarose gel and isolated by a DNA purification kit (Jetsorb, Genomed, Bad Oeynhausen, Germany). Sequencing of PCR fragments was performed with the Taq Dyedeoxy Terminator Cycle Sequencing, kit (Applied Biosystems, Foster City, California, USA). Sequencing, reactions were resolved on an ABI 373A automated sequencer. Positions of introns have been determined by predicted splice donor or splice acceptor sites.

### Mutation detection

A combined SSCP (Orita *et al*. (1989) *Ann. Hum. Genet.* 59: 347-365) and heteroduplex analysis (White *et al*. (1992) *Genomics*, 12: 301-306) approach was used using optimized conditions (Glavac and Dean (1993) *Hum. Mutation*, 2: 404-414). DNA samples (100 ng) were amplified in PCR buffer containing, 1.5 mM MgCl, and ³²P-dCTP for 35 cycles of 94°C, 30 sec, 55°C, 30 sec, 72°C, 30 sec. Products were diluted 1:3 in stop solution, denatured at 95°C for 2 min and 3 µl loaded directly on gels. Gel formulations used were 1) 6% acrylamide:Bis (2.6% crosslinking,), 10% glycerol, room temp, 45W; 2) 6% acrylamide:Bis (2.6% crosslinking), 4 60W; 3) 10 % acrylamide:Bis (1.3% crosslinking) 10% glycerol4, 60W; 4) 0.5X MDE (ATGC Corp, Malvern, PA), 10% glyrerol4, 50W. Gels were run for 3-16 hours(3000Vh/100 bp), dried and exposed to X-ray film for 2-24 hrs. Heteroduplexes were identified from the double-stranded DNA at the bottom of the gels, and SSCPs from the single-stranded region.

Samples showing variation were compared to other family members to assess segregation of the alleles, or to normal DNA from the same patient, in the case of tumors. PCR products with SSCP or heteroduplex variants were treated with shrimp alkaline phosphatase and exonuclease I (United States Biochemical) and cycle sequenced with AmplitaqFS^{™} (Perkin Elmer, Norwalk, Connecticut, USA). The products were analyzed on an Applied Biosystems model 373 DNA sequencer.

### Results and Discussion

### Fine Mapping by Linkage and Tumor Deletion Studies

Since the original mapping of the gene in 1992, linkage studies have narrowed the *NBCCS* region to a 4 cM interval between *D9S180* and *D9S196* (Goldstein *et al*. (1994) *supra*; Wicking *et al.* (1994) *Genomics*, 22: 505-511). Farndon *et al.* (1994) *supra,* reported recombination involving an unaffected individual that tentatively placed the gene proximal to *D9S287*.

The present experiments identified one recombination between *D9S287* and *FACC* in a three-generation family. The recombinant individual was a 1.5 year old female presumed to be affected on the basis of macrocephaly, strabismus, and frontal bossing. Some of the key features of the syndrome such as basal cell carcinomas, jaw cysts, and palmar pits, were lacking; but these features have age-dependent expression, and their presence in a young child would not be expected. With the assumption that she carried the gene, the recombination in this family placed *NBCCS* proximal to *D9S287*.

Allelic loss in BCCs was concordant with linkage mapping in placing the gene between D9S 196 and D9S180. Most hereditary tumors with allelic loss deleted the entire region between the flanking, markers. However, one hereditary cardiac fibroma showed loss at D9S287 but not D9S280 on the non-disease-carrying, allele suggesting that the gene is located distal to D9S280. In sporadic BCCs four tumors were found that retained D9S287 and lost more distal markers but also two tumors that lost the proximal marker D9S280, but not D9S287.

Several hypotheses can be proposed to explain this discrepancy in tumor deletion mapping, and between tumor deletions and linkage studies. A gene other than NBCCS could be responsible for the allelic loss in some sporadic tumors. NBCCS is almost certainly the target of allelic loss in hereditary tumors because these tumors always lose the copy of the NBCCS gene from the unaffected parent and retain the inherited mutation (Bonifas *et al.* (1994) *Hum. Mol. Genet*., 3: 447-448). If a second locus were driving allelic loss, then the alleles from the affected parent and the unaffected parent would be lost with equal frequency. However, there may be two different tumor suppressors on chromosome 9q that are both important in basal cell carcinomas. The APC gene and MCC gene, for example, are both mutated in colon cancer and lie within 1 Mb of each other on chromosome 5q (Hampton *et al.* (1992) *Proc. Natl. Acad. Sci. USA* 89: 8249-8253). The observation of a clearly distinct pattern of allelic loss, involving, *D9S180* but not *D9S287*, in squamous cell carcinoma of the skin supports the presence of more than one tumor suppressor in the 9q22.3 region. Additionally a putative tumor suppressor has been mapped to 9q21-31 in bladder cancers, and may be distinct from the NBCCS gene (Knowles *et al*. (1995) *Br. J. Urol*. 75: 57-66).

A second possibility is that regions on both sides of *D9S287* are deleted in some tumors, but that the more proximal deletions are not always detected by available markers. In fact two tumors were observed that deleted markers both proximal and distal to *D9S127,* probably reflecting genetic instability in tumor cells. Finally, NBCCS could be a large gene that extends on both sides of the *D9S287* locus. Taken together the data provided herein suggested that the most likely location of the NBCCS gene was between markers *D9S280* and *D9S287.* Nevertheless, due to the discrepancies in tumor deletions, physical mapping and cDNA isolation from the entire region between *D9S196 and D9S180* were undertaken.

### Physical mapping

Twenty-nine YACs containing markers from this region were obtained from the CEPH megaYAC library. Eighteen formed an overlapping contig between *D9S196* and *D9S180* with at least 2-fold redundancy. Based on this contig the minimum distance between the flanking markers was 1.5 Mb, but virtually all large YACs had internal deletions as judged by STS content. Additional YACs were obtained from the ICI library to provide redundancy in areas apparently prone to deletion. Cosmid and BAC contigs were constructed around known STSs and genes, and additional cosmids from the region were isolated by hybridizing YACs to the Lawrence Livermore gridded cosmid library. In total over 800 cosmids specific to this region were gridded into 96-well grid plates and contigs of BACS, P1s and cosmids covering 1.5 Mb were constructed (Figure 1). Because of deletions in YACs and some gaps in the cosmid and BAC contig, pulsed field gel electrophoresis (PFGE) and FISH were used to integrate the cloned regions. Based on the sizes of restriction fragments in this region and FISH estimates, the physical distance from *D9S180 to D9S196* was estimated at not less than 2 Mb.

### Isolation of cDNAs

Harshman *et al.* (1995), *supra,* showed that different methods of identifying cDNAs from a genomic region result in a surprisingly different array of candidate genes. Several methods were used to find genes that map to chromosome 9q22 including sample sequencing of cosmids, exon trapping, HTF island cloning, and direct selection of cDNAs from BACs and cosmids. In addition, genes known to lie in this general area were more finely mapped by use of somatic cell hybrids made from two NBCCS patients with visible 9q22 deletions (submitted to NIGMS repository), YAC contigs, and FISH. Ten genes, ten ESTs with sequences in GENBANK, and 31 anonymous selected cDNA fragments, HTF island clones, and trapped exons with no known homology were identified (Table 1).

### Screening patients for germline deletions or rearrangements

Because chromosome 9q22 appeared to be very gene rich, an attempt was made to localize the *NBCCS* gene more precisely by searching for submicroscopic rearrangements in patients. Fifteen cosmids at approximately 100-200 kb intervals spanning the region between *D9S196* and *D9S180* were hybridized to PFGE blots of 82 unrelated NBCCS patients. In addition probes from genes known to map to the interval as well as those identified in the course of the study were included in this analysis. PFGE variants were identified in three patients with genomic probes from within the Fanconi's anemia complementation group C (*FACC*) gene. All three were heterozygous for SaclI bands approximately 30 kb shorter than normal (310 vs 280 kb). The limit of resolution of PFGE was about 10 kb, so that it was not possible to determine whether the apparently identical variant SacII bands were exactly the same size. Other restriction enzymes including Notl, BssHII, MluI, Sfil, and NruI did not show variant bands. The variations were not consistent with germline deletions in these patients but could conceivably be caused by point mutations creating new restriction sites or other small alterations such as the recurrent inversions seen in the F8C gene of many hemophiliacs (Lakich *et al.* (1993) *Nat. Genetics,* 5: 236-241).

The nature of the DNA alterations causing these changes on PFGE has not yet been elucidated, but the data relating them to the disease are compelling. The families of two of the patients with variations were not available for study. However the third patient was a sporadic case of NBCCS, and neither parent had the SacII alteration. The finding of this variant in a patient but not in her parents could be interpreted as the result of hypermutability of some CG-rich region near *FACC*, but no variation in this region was identified in PFGE blots of over 100 normal chromosomes.

**Table 1. cDNA clones from the NBCCS region on chromosome 9q22.3.**

| Clone Designation^{a} | Clone type^{b} |
|---|---|
| *cDNAs previously mapped to chromosome 9q and more finely mapped with somatic cell hybrids, PFGE, and YAC contigs.* | |
| FACC | Gene |
| NCBP | Gene |
| HSD17B3 | Gene |
| TMOD | Gene |
| XPA | Gene |
| *SYK* | Gene |
| *WI-11139* | EST (R14225) |
| *WI-11414* | EST (T88697) |
| *WI-8684* | EST (R14413) |
| D9S1697 | EST (R06574) |
| D9S1145 | EST (contains R17127 and Z38405) |

| *Novel clones or clones not previously mapped to chromosome 9q identified by sample sequencing, exon trapping, HTF island cloning or cDNA selection.* | |
|---|---|
| *ZNF169* | Gene |
| *FBP1* | Gene |
| *PTC* | Gene |
| *Coronin* homologue | Gene |
| *2F1a* | EST (R39928) |
| *2F1b* | EST (T11435) |
| *11F21* | EST (Z43835) |
| *31F3* | EST (R16281) |
| *yo20g05.s1* | EST (Merck EST) |
| *plus 31 anonymous selected cDNA fragments, HTF island clones, and trapped exons from YA Cs and cosmid pools*^{c} | |

| | |
|---|---|
| ^{a}For known genes and anonymous cDNAs that have been submitted to the Genome Data Base (http://gdbwww.dgw.org/gdb), standard locus nomenclature is used. For ESTs without a GDB number, WI indicates a Whitehead Institute clone (http://www-genome.wi.mit.edu). ^{b}NCHR accession numbers, where available, are given in parentheses for anonymous ESTs. Additional information can be obtained at http://www.ncgr.org/gsdb. ^{c}Additional sequence data will be required to determine whether some of the anonymous cDNAs represent different portions of the same genes. | |

### Evaluation of PTC as a candidate gene

Because variant PFGE bands were identified in the *FACC* region and one recombinant as well as tumor deletion studies suggested a possible location near this marker, candidate cDNAs that mapped to this area were examined. *FACC*, itself, was not considered as a candidate because heterozygous mutations in this gene do not cause NBCCS (Strathdee *et al*. (1992) *Nature,* 356: 763-767). *FACC and PTC* (a novel human gene with strong homology to *Drosophila patched*) hybridized to the same 650 kb Notl fragment and 675 kb and 1000 kb (partial) MluI fragments. Mouse interspecies backcross analysis determined that there were no recombinants between the *PTC and FACC* genes out of 190 meioses. *PTC and D9S287* were both present on ICI YAC 2EF8 having, a size of 350 kb strongly suggesting, that *PTC* lies between *D9S287 and FACC*.

To screen for mutations in the *PTC* gene, the intron/exon boundaries of the gene were determined from genomic clones and long range PCR products. *PTC* consists of 21 exons and the gene spans approximately 34 kb (Figure 2). Panels of unrelated NBCCS patients and BCCs were screened by single-stranded conformation polymorphism (SSCP) analysis (primers used for amplification of *PTC* exons are shown in Table 2). Patients displaying variations were compared to unaffected individuals of the same race, and variants found only in affected individuals were further characterized by DNA sequencing. Of the mutations identified in unrelated patients, four were deletions or insertions resulting in frameshifts and two were point mutations leading to premature stops (Table 3, Figures 4 and 5). An additional finding, confirming the relationship between mutations in *PTC* and the disease was identification of a frameshift mutation in a sporadic NBCCS patient that was not present in either of her unaffected parents (Figure 5).

To analyze the role of *PTC* in neoplasia, tumors related to the syndrome were screened for mutations. Two sporadic basal cell carcinomas with allelic loss of the NBCCS region had inactivating mutations of the remaining allele (Figures 6 and 7). A tumor removed from the cheek had a CC to TT alteration, typical of UVB mutagenesis. The second tumor from the nose had a 14 bp deletion, a mutation that cannot be related to any specific environmental accident. Mutations have not yet been identified in any sporadic BCC not showing allelic loss of chromosome 9q22, and alternative modes of pathogenesis may be operative in these neoplasms.

**Table 3. Mutations in the PTC gene.**

| Sample Type | Inheritance | Exon | Type of Mutation | Designation |
|---|---|---|---|---|
| NBCCS | F | 5 | premature stop | C1081T |
| NBCCS | F | 6 | 37 bp deletion | del 804-840 |
| NBCCS | F | 8 | premature stop | G1148A |
| NBCCS | F | 12 | 2 bp insertion | 2047insCT |
| NBCCS | S | 12 | 1 bp insertion | 2000insC |
| NBCCS | S | 14 | 1 bp deletion | 2583delC |
| BCC | S | 5 | premature stop | CC1081TT |
| BCC | S | 15 | 14 bp deletion | del 2704-2717 |

| | | | | |
|---|---|---|---|---|
| ^{a}NBCCS, germline mutations in a patient with the syndrome; BCC, somatic mutation in a basal cell carcinoma. ^{b}F, familial; S, sporadic. | | | | |

### Discussion

These examples provide strong evidence that mutations of the NBCCS gene (*PTC*)*,* the human homologue of *Drosophila patched* cause the nevoid basal cell carcinoma syndrome. Alterations predicted to inactivate the *PTC* gene product were found in six unrelated NBCCS patients. Frameshift mutations were found in two sporadic patients but not in their parents, and somatic mutations were identified in two sporadic tumors of the types seen in the syndrome. No known human tumor suppressor has sequence similarity to *PTC,* and functionally *PTC* may represent a novel type of neoplasia-related gene.

### The Drosophila patched gene in differentiation and development

The *patched* gene is part of a signaling pathway that is conserved from flies to mammals. The *Drosophila* gene *(ptc)* encodes a transmembrane glycoprotein that plays a role in segment polarity (Hooper and Scott (1989) *Cell* 59: 751-765; Nakano *et al*. (1989) *Nature* 341: 508-513). Many alleles of *ptc* produce an embryonic lethal phenotype with mirror-image duplication of segment boundaries and deletion of the remainder of the segments (Nusslein-Volhard *et al*. (1980) *Nature* 287: 795-801), but hypomorphic alleles produce viable adults with overgrowth of the anterior compartment of the wing, loss of costal structures, and wing vein defects (Phillips *et al*. (1990) *Development* 110: 105-114). Genetic and functional studies have shown that one of the wild type functions of *ptc* is transcriptional repression of members of the *Wnt* and *TGF-b* gene families (Ingham *et al*. (1991) *Curr. Opinion Genet. Develop.* 5: 492-498; Capdevila *et al*. (1994) *EMBO J.* 13: 71-82. The mechanism of this repression is not known, and many other downstream targets of *ptc* activity may exist.

The action of *ptc* is opposed by the action of members of the *hedgehog* gene family. Studies in *Drosophila* have demonstrated that *hedgehog (hh)* is a secreted glycoprotein which acts to transcriptionally activate both ptc-repressible genes and *ptc* itself (Tabata and Kornbernc (1994) *Cell,* 76: 89-102; Basler and Struhl (1994) *Nature,* 368: 208-214). Thus, in a given cell type the activity of target genes results from a balance between hedgehog signaling, from adjacent cells and *ptc* activation.

### Mammalian homologues of patched

The human homologue *(PTC)* of *Drosophila ptc* of this invention, displays no more than 67% identity at the nucleotide level and 61 % identity at the amino acid level to the *Drosophila* gene. Thus identification of a human homolog would have been difficult using either a hybridization approach or by screening, an expression library with antibodies to the fly protein. The present data strongly suggest that *patched* is a single copy gene in mammals.

Analysis of fetal brain cDNA clones, and RACE experiments with epidermal RNA revealed the presence of two different 5' ends for the human *PTC* gene (Figure 3). The two human sequences diverge from the mouse *PTC* cDNA (from 8 dpc embryo RNA) at the same position, and the mouse N-terminus more closely matches the *Drosophila* and *C. elegans* proteins. Analysis of the upstream genomic sequence of the *C. elegans* gene failed to reveal any homology to the two alternate human ends. These data suggest that there are at least three different forms of the *PTC* protein in mammalian cells the ancestral form represented by the murine sequence, and the two human forms. The first in-frame methionine codon for one of the human forms is in the 3rd exon, suggesting that this form of the mRNA either encodes an N-terminally truncated protein, or uses an alternate initiation codon. The second human form contains an open reading frame that extends through to the 5' end and may be initiated by upstream sequences that have not yet been isolated. The identification of several potential forms of the PTC protein provides a mechanism whereby a single PTC gene could feasibly play a role in different pathways. It will be important to determine the regulation of the different splice forms of *Ptc* mRNA as this may shed light on the apparent role of the gene in both embryonic development and growth control of adult cells.

In adult humans *PTC* is expressed widely. Abundant transcript is found in the kidney, liver, lung, brain, heart, skeletal muscle, pancreas, and skin. During murine development *Ptc* is expressed first at 8.0 dpc in ventral neuroepithelial tissue in two separate domains along the midline. By day 9.5, transcripts are detected in the mesenchyme surrounding the neural tube as well. Expression is seen in the developing somites in a rostral-caudal gradient. From day 10.0 to 12.5 the transcript is present in the posterior ectoderm of each limb bud. Other sites of expression during this period include the inner surfaces of the pharyngeal arches, cells surrounding the placodes of the vibrissae, and the genital eminence.

Several homologues of *Drosophila hedgehog (hh)* have been identified in vertebrates and, like the Drosophila gene, appear to be involved in pattern organization during development. The most extensively studied of these is *Sonic hedgehog (Shh)*. In the mouse, expression of *Shh* is normally detected in the notochord and the overlying floorplate region of the neural tube (Echelard *et al*. (1993) *Cell* 75: 1417-30). Apart from being involved in midline signaling in vertebrates, Shh is also expressed in a number of other tissues, including the developing limbs, where it appears that *Shh* normally mediates the activity of the zone of polarizing activity (ZPA).

The expression of murine *Ptc* is found in a variety of tissues known to be responsive to *Shh* signaling. A detailed expression analysis has indicated that the pattern of expression closely follows changes in *Shh* expression such that the transcripts are found mostly in adjacent, non-overlapping tissues. *Ptc* may be required for *Shh* signaling, and *hh*/*ptc* interactions appear to have been conserved during evolution. As in flies, *Ptc* transcription in mouse appears to be indicative of an adjacent *hh* signal. Accordingly, when interpreting the relationship between known sites of *Ptc* expression and the NBCCS phenotype, it may be of value to consider the expression pattern of *hedgehog* gene family members since they have been characterized in much more detail in vertebrates than ptc, especially in adult tissues.

While it is clear that genetically and functionally *Ptc* responds to *hh* signaling, its structure does not make it an obvious *hh* receptor. Rather, it has been proposed that *Ptc* may be a transporter and the substrates are molecules which regulate the transcription of target genes.

### The role of PTC in neoplasia

The data presented in this study strongly suggest the *NBCCS* gene functions as a tumor suppressor. These examples show that germline mutations underlying the NBCCS phenotype are inactivating, and therefore hereditary tumors have no functional copy of the gene. In addition, these examples provide the first direct evidence that sporadic basal cell carcinomas (BCCs) can arise with somatic loss of both copies of the gene. The role *of PTC* in other tumors related to the syndrome remains to be explored.

That two known targets of *ptc* repression in *Drosophila* represent gene families involved in cell-cell communication and cell signaling provides a possible mechanism by which *ptc* could function as a tumor suppressor. The *ptc* pathway has recently been implicated in tumorigenesis by the cloning, of the pancreatic tumor suppressor gene, *DPC4* (Hahn *et al.* (1996) *Science* 271: 350-353), which shows sequence similarity to Drosophila *mad* (mothers against *dpp*). The *mad* gene interacts with *dpp*, *a Drosophila TGF-b* homologue specifically repressed by *ptc*.

The cell of origin of BCC has been greatly debated and current theory postulates a progenitor "stem cell" that is slow cycling, but of great proliferative potential (Miller (1991) *J. Am. Acad. Dermatol.* 24: 161-175). Through occasional cell division "transient amplifying cells" are formed, which further multiply before committing to terminal differentiation. The expression of *patched* and *hedgehog* gene family members in skin is not known. In *Drosophila, ptc* is found in membrane regions resembling cell adhesive junctions, and it colocalizes with PS2 integrins, suggesting that the human homolog may normally play a role in epidermal differentiation based upon cell/cell interactions. The correct localisation of Drosophila *ptc* protein to these regions is also dependent upon the interaction of the cells in a polarized epithelial sheet, an observation also supporting a role for *ptc* in cell adhesive structures (Capdevila *et al.* (1994) *Development* 120: 987-998). The present finding suggest that BCCs lack intracellular PTC signaling leading to an overexpression of the proliferative and cell/cell communication genes associated with *hedgehog* signaling. Alternatively, if PTC has a direct role in cell/cell interactions, proliferation may result from disruption at this level.

### The role of PTC in developmental anomalies

By analogy to embryonic expression of *Ptc* in the mouse, many of the features of NBCCS can be correlated with the presumed sites of expression of *PTC* in the developing human embryo (Table 4). For example, the skeletal anomalies involving the ribs, vertebrae and shoulders are most likely due to disruption of PTC expression in the sclerotome. In the mouse embryo *Ptc* expression is detected in the ventral-medial cells of the somites, a region which subsequently forms sclerotome (Hahn *et al.* (1996) *supra.;* Goodrich *et al.* (1996) *Genes Dev.* 10: 301-12). In addition, *Shh* has been implicated in the induction of sclerotome by long range signaling from the notochord (Fan *et al.* (1995) *Cell* 81: 457-465).

**Table 4. Mouse Ptc expression and human NBCCS phenotype.**

| Site of expression in mouse | NBCCS Phenotype |
|---|---|
| Pharyngeal arches | Facial malformations J |
| | aw cysts (dental lamina derivative |
| | |
| Neural tube | Dysgenesis of the corpus callosum |
| | Eye anomalies |
| | |
| Somites | Spina bifida |
| | Vertebral fusion |
| | Rib anomalies |
| | |
| Limb buds | Short fourth metacarpals |
| | Polydactyly |

The polydactyly observed in a subset of NBCCS patients is likely to correlate with the expression of *Ptc* in the developing murine limb (Hahn *et al*. (1996) *supra*.; Goodrich *et al.* (1996) *supra.).* While the actual mechanisms remain unknown it seems clear that anterior posterior patterning of the limb is controlled by *Shh* signaling from the ZPA. In the early mouse limb bud *Ptc* expression correlates with *Shh* while at later stages expression is detected in the periphery of the digital condensations in cells adjacent to those expressing Indian hedgehog (*Ihh*) (Goodrich *et al.* (1996) *supra*.) Therefore the polydactyly present in NBCCS may result from perturbation of limb patterning due to modulation of *PTC*. Similarly the occurrence of immobile thumbs in a small percentage of NBCCS patients is consistent with an alteration to wild type *PTC* function.

Craniofacial dysmorphology correlates with expression of *Ptc* in the pharyngeal arches and derived structures. The jaw keratocysts and dental malformations, which are common features of NBCCS, are most likely explained by the observed expression of *Ptc* in the tooth bud and the enamel knot (Vaahtokari *et al*. (1996) *MOD, 54:* 39-43; Goodrich *et al.* (1996) *supra*.). The pathogenesis of jaw cysts is almost certainly related to the embryologic dental precursors. The epithelial lining of keratocysts is believed to arise from aberrant derivatives of the dental lamina, the precursor of tooth buds. The progenitor cells may have migrated abnormally during development of the lamina or failed to involute at the appropriate stage of development.

The neurological components of NBCCS such as agenesis of the corpus collosum, retinal colobomas, and possibly strabismus and macrocephaly are consistent with the expression of *Ptc* in the developing brain and neural system. Mental retardation, seen occasionally in the syndrome, may be caused by contiguous gene deletions.

Under the classical two-hit model for the action of tumor suppressors (Knudson (1971) *Proc. Natl. Acad. Sci. USA*, 68: 820-823) the finding of developmental defects in a syndrome caused by hemizygous inactivation of this type of gene constitutes a paradox because loss of just one copy is thought to have little or no effect on cell function. It is possible that some of the discrete defects in NBCCS (*e*.*g*., spina bifida occulta, bifid ribs, and jaw cysts) can be explained by a two-hit mechanism. Like the neoplasms in cancer predisposition syndromes many of these defects are multiple and appear in a random pattern, but isolated defects of the same type are seen occasionally in the general population. These anomalies might result from homozygous inactivation of *PTC* in an early progenitor cell of the relevant tissue leading to abnormal migration or differentiation or perhaps failure to undergo programmed cell death. Allelic loss studies have in fact shown that keratocysts of the jaw are clonal abnormalities that arise with homozygous inactivation of the *NBCCS* gene (Levanat *et al*. (1996) *Nat. Genetics* 12: 85-87). However, generalized or symmetric features such as overgrowth, macrocephaly, and facial dysmorphology almost certainly defy the two-hit paradigm and are probably due to haploinsufficiency. It appears that many of the developmental defects seen in NBCCS patients result from perturbation of a dosage sensitive pathway during embryonic development. Based upon these observations one prediction would be that a heterozygous *Ptc* "knock-out" mouse would show relatively mild developmental anomalies and UV irradiation of the skin would result in multiple basal cell carcinomas.

### Phenotypic variation in NBCCS

NBCCS is a disorder with almost 100% penetrance, but many of the features show variable expression. An interesting correlate in *Drosophila* is that flies homozygous for non-lethal alleles show striking variability in expression of phenotypic features. Because these flies are isogenic, the phenotypic differences cannot be explained by different underlying, mutations or modifying genes (Phillips *et al.* (1990) *supra*.*)*. Presumably the variability is a stochastic effect.

That there is more similarity in the human NBCCS phenotype within families than between families (Anderson *et al*. (1967) *supra*.) suggests that there may be some degree of genotype/phenotype correlation. At present there are no clearly defined patterns of mutation distribution in NBCCS and all mutations found so far are predicted to cause truncation of the *PTC* protein. In families with BRCA 1 termination mutations, ovarian cancer is more common in patients with 5' mutations, perhaps because mutant peptides may retain some wild-type function in some cell types but not in others (Gayther *et al.* (1995) *Nature Genetics* 11: 428-433).

### Example 3

### Characterization of PTCH Germ Line Mutations in NBCCS

### Materials and Methods

### DNA extraction

Odontogenic keratocyst tissue was placed in 200 µl STE buffer (50mM NaCl; 10mM Tris-HCI pH8.0; 1mM EDTA) containing 0.5% w/v SDS, 1µg/µl proteinase K and incubated at 37°C for 24 hours. Following inactivation of the proteinase K at 95°C for 15 minutes, a sample of 1-5 µl (approximately 25ng DNA) was used directly for PCR. Constitutional DNA was obtained from peripheral blood lymphocytes or buccal epithelial cells using standard DNA extraction procedures. Approximately 25ng DNA was used directly for PCR.

### Genbank Accession Numbers

DNA sequence data for the PTCH gene are available under accession numbers U43148 and U59464. The nucleotide numbering used in this Example corresponds to sequence U43148, whereas the amino acid residue numbering corresponds to U59464.

### PCR-SSCP analysis of the PTCH gene

Each of the 23 exons comprising the patched gene were amplified separately using the primers and annealing conditions described in Hahn *et al*. (1996), *supra.* In brief, approximately 25ng target DNA was amplified in 30 µl 1xPCR reaction buffer containing 50mM KCl; 10mM Tris-HCI (pH 9.0); 1.5mM MgCl₂; 0.1% v/v Triton X-100; 200µM each dATP, dGTP, dTTP, 20 µM dCTP; 10 picomoles of each primer; 1.0µ Ci [α³²P-dCTP] and 1.0 unit Taq. DNA polymerase (Promega, UK). Following 35 cycles of amplification, 5µl PCR product was added to 35µL 10mM EDTA, 0.1% w/v SDS. 2µl of this was added to 2 µl loading buffer containing 95% v/v deionised formamide/20mM EDTA/0.05% w/v bromophenol blue/0.05% w/v xylene cyanol, heated to 100°C for 5 minutes, quenched on ice and loaded onto a 1xTBE/6% w/v non-denaturing polyacrylamide gel (5%C) containing (5%C) containing 5% v/v glycerol. Electrophoresis was at 350V for 18 hours. Gels were dried under vacuum and autoradiographed for 16 hours at room temperature with intensifying screens.

### Restriction SSCP

PCR products obtained by amplification of exons 14 and 17 were restriction enzyme digested with *Alul* and *Hinfl*, respectively, prior to SSCP analysis. An aliquot of *10* µl PCR product was digested in a total volume of 20 µl 1X reaction buffer according to manufacturers' instructions. 2 µl of restriction enzyme digested PCR product was mixed with 2 µl loading buffer and subjected to SSCP analysis as described above.

### DNA sequencing of PTCH exons

Exonic PCR products displaying altered mobilities by SSCP analysis were purified using commercially available columns (Wizard^{™} PCR columns, Promega). PCR products were eluted in 20 µl TE and 2 µl used for DNA Thermosequenase^{™} (Amersham International plc) cycle sequencing according to manufacturer's instructions. International plc) cycle sequencing according to manufacturer's instructions. Sequencing primers were end-labelled with γ³²P-ATP (3000 Ci/mmol) using T4 polynucleotide kinase. DNA sequencing reactions were fractionated in 6% w/v polyacrylamide/8M urea/1xTBE gels for 2 hours at 2000V. Gels were dried under vacuum and autoradiographed for 8 hours at room temperature with intensifying screens.

### Results

Keratocyst DNA from a total of 16 NBCCS patients was screened by SSCP-PCR. 10 single exonic PCR products displaying altered electrophoretic mobilities were detected and analyzed further by DNA sequence analysis. In addition, variant bands in multiple samples (*i*.*e*. indicative of common polymorphisms) were also seen in PCR products encompassing 4 exons.

Four mutations were identified following direct DNA sequencing of PCR amplified exons displaying SSCP variant bands. Mutations could not be detected in the remaining 6 variant PCR products. Therefore, all 23 exons from the samples in which a mutation had not been identified initially were amplified and sequenced in their entirety. However, only one additional mutation was detected by this method.

### Exon 5 693 insC

A single cytosine residue insertion at position 693 was detected in a 42 year old male NBCCS patient. This introduces a frameshift mutation by the creation of a premature stop codon at amino acid residue 252. This mutation also creates a *BstN*I restriction enzyme site. DNA from the patient and 4 unaffected family members was amplified and restriction enzyme digested with *BstNI*. As predicted, only the product from the NBCCS patient was cut by the restriction enzyme.

### Exon 17 2988 del8bp

Following *Hinf*I restriction enzyme digestion of exon 17 PCR products and SSCP analysis, 2 variant bands were seen. Direct DNA cycle sequencing of these amplicons revealed 2 mutations. An 8bp deletion was detected in DNA from a 12 year old male NBCCS patient. This frameshift mutation introduces a stop codon at amino acid residue 1141. The patient has macroephaly, hypertelorism, supra-orbital ridges, prognathism, plantar but not palmar pitting and an accessory nipple. In addition, at age 10 years, the patient had undergone surgical removal of 3 maxiallary and mandibular odontogenic keratocysts.

### Exon 17 3014 insA

An adenosine insertion at base 3014 was detected in an 18 year old female NBCCS patient. This results in a frameshift mutation (tyrosine to STOP) codon at amino acid residue 1009. This patient was frontal bossing, hypertelorism, falx calcification, bifid 3rd, 4th, 5th and 6th ribs and has undergone enucleation of 5 maxillary and mandibular odontogenic keratocysts.

### Exon 21 3538 delG

A guanosine base deletion at residue 3538 was identified in a 38 year old female NBCCS patient. This frameshift mutation introduces a stop codon at amino acid residue 1190. The causal nature of the mutation was confirmed by analysis of DNA from the proband's father, from whom she has inherited the disorder. Direct DNA sequencing of exon 21 from the father also revealed a guanosine base deletion at residue 3538.

### Exon 22 G4302T

Direct DNA sequencing of 23 exons from a 30 year old female NBCCS patient revealed a G-T substitution at nucleotide 4302. This causes a glutamic acid to aspartic acid (E - D) substitution at amino acid residue 1438. The patient has been confirmed as a case of NBCCS and has undergone removal of 11 basal cell carcinomas.

### PTCH gene polymorphisms

Using the SSCP conditions described, the inventors observed polymorphisms in PCR products amplified from exons 6, 11, 14 and 15. No DNA sequence alterations were detected following analysis of exonic sequences. Therefore, it is likely that these represent intronic DNA sequence polymorphisms. Also, an exonic DNA sequence polymorphism (C306T) was disclosed in exon 2. This base substitution was observed in DNA from subject LDI-1, in which a "causative" 3538delG mutation had already been identified, as well as other unrelated NBCCS patients.

In this example, the inventors identified 5 novel, germ line mutations from patients with the NBCC cell, consistent with the role of patched as a human tumor suppressor gene. Four mutations cause frame-shift or non-sense mutations resulting in a truncated PTCH protein; the fifth mutation is a glutamic acid to aspartic acid substitution close to the 3'-carboxyl terminus of the PTCH protein. This was the only base change detected following direct DNA sequencing of all 23 PTCH exons from patient #5. Although this represents a conservative amino acid substitution and, therefore, may be a polymorphism and not a mutation, this glutamic acid residue is conserved between human, mouse and chicken PTCH proteins and is likely to be functionally important.

### Example 4

### Mutations of the PTCH Gene in NBCCS Define Clinical Phenotype

Example 3 defined mutation in individuals with NBCC syndrome. In this Example, further mutations are identified.

### Materials and Methods

The patients were diagnosed according to the clinical criteria of Shanley *et al* (1994). Seventy NBCCS patients were fully analyzed by single strand conformation polymorphism (SSCP) and heteroduplex analysis as previously described (Hahn *et al., supra*.) with primers for all coding exons except for exon 1b (alternative first exon). Primer sequences were as hereinbefore described as well as Hahn *et al* (1996) with the exception of exons 12, 12b and 20. Exon 12b is an additional exon resulting from the discovery that exon 12 (Hahn *et al.* 1996) consists of two distinct exons. The *PTC* gene, therefore, consists of 23 coding exons. Where possible, DNA was also analyzed from the parents of cases in which *PTC* mutations were found in order to determine at a molecular level whether the mutation was sporadic or familial. Any samples showing SSCP variants were sequenced as previously described (Wicking *et al.* (1997) *Am. J. Hum. Genet.* 60: 21-26).

Paternity testing was carried out using microsatellite markers in the parents of the eight sporadic cases using fluorescent primers and Genescan.

### Results

*PTCH* mutations were identified in a total of 32 NBCCS cases. Twenty-eight of these are described in Example 3. This Example presents four novel mutations (Table 5). Of these, three are frameshift mutations and one is a putative splice variant. The inventors have, therefore, detected PTCH mutations in 32/70 (46%) NBCCS cases by analysis of all exons except exon 1b. The majority of these (27/32; 84%) are protein terminating mutations. In addition, eight sporadic cases were identified by the absence of the relevant disease-associated mutation in either parent (Table 6). Paternity testing was performed in these eight cases, using four microsatellite markers and in none was any inconsistency identified.

This Example presents eight individuals with NBCCS whom the inventors have shown to carry mutations in the *PTCH* gene. In all cases, the parent did not carry the disease-related mutation, and non-paternity was shown to be unlikely by analysis of highly informative microsatellite markers. In addition, the inventors report four NBCCS individuals with germline *PTCH* mutations. Three out of four of these would result in premature protein truncation.

The ability to confirm the diagnostic status of relatives of NBCCS cause by DNA analysis allows further definition of the clinical and radiological criteria used to diagnose NBCCS. Of the eight NBCCS individuals shown to have new mutations in the *PTCH* gene in this study only two (JRN250, DD25) clearly represented sporadic cases based on clinical and radiological examination of both parents. In one case (JHK551) neither parent had been examined. In all other cases one parent showed at least one feature associated with NBCCS such as multiple BCCS, a high arched palate or macroephaly.

**TABLE 5**

| ***PTCH* MUTATIONS IN NBCCS INDIVIDUALS** | | | |
|---|---|---|---|
| **Patient** | **Exon** | **Mutation** | **Effect on coding** |
| JK211 | 12 | 1711insC | Frameshift, truncation |
| MB229 | 12 | 1639insA | Frameshift, truncation |
| CW424 | 16 | 2707delC | Frameshift, truncation |
| NB88 | Intron 17 | 3157-2A-> G | Putative splice variant |

**TABLE 6**

| **MUTATIONS OF PTCH IN NBCCS PATIENTS** | | | |
|---|---|---|---|
| **Patient** | **Mutation** | **Effect** | **Parental phenotype** |
| DD25 | C2050T | Nonsense | Parents both clinically and radiologically negative but multiple BCCs in history of grandmother. |
| JHG547 | C391T | Nonsense | Father has had > 10 BCCs from 6th decade but radiologically negative. Mother clinically and radiologically negative. |
| BK273 | 244delCT | Frameshift | Father has high arched palate, macrocephaly and dense falcine calcification (age 53) but below maximum biparietal diameter. The Mother clinically and radiologically negative. |
| JHK551 | 271 insA | Frameshift | Negative family history but neither parent examined. |
| JRN250 | 929delC | Frameshift | Both parents clinically and radiologically negative. |
| MP264 | 2183delTC | Frameshift | Father has high arched palate, macrocephaly and three "pits" on soles but radiologically negative. Mother clinically and radiologically negative. |
| KS356 | 2583delC | Frameshift | Father has had three unconfirmed BCCs (age 65) but radiologically negative. Mother negative clinically. No radiological examinations. |
| JK211 | 1711insC | Frameshift | Father has high arched palate and has had about 20 BCCs (from age 70) but also multiple solar keratoses, keratoacanthoma and squmous cell carcinomas. He is radiologically negative. Mother clinically and radiologically negative. |

### Example 5

### Medulloblastomas of the Desmoplastic Variant carry Mutation of the Human Ptc Gene

In this Example, the inventors detected non-conservative *PTC* mutations in three of 11 sporadic cases of desmoplastic medulloblastomas (Mbs) but none in 57 tumours with classical (non-desmoplastic) histology. In two of the tumours with mutations and in two additional desmoplastic cases, LOH was found at 9q22. These findings suggest that *PTC* represents a tumour suppressor gene involved in the development of the desmoplastic variant of MB.

### Materials and Methods

### Patient and tumours, cell lines.

A total of 68 medulloblastoma samples were analyzed, 64 samples were obtained from MB tumors and 4 from the previously described medulloblastoma cell lines D283Med, D341Med, Daoy, and MHH-MED-1 (Pietsch *et al.* (1994) *Cancer Res.* 54: 3278-3287). In two patients, the inventors were able to study both the primary and the recurrent tumors. Constitutional DNA from peripheral blood was available in 40 patients. DNA samples from peripheral blood from healthy Caucasian volunteers were used as controls. A sample of normal cerebellum was analyzed. This biopsy specimen was from an adult patient with a cerebellar vascular malformation and was found to be normal upon histopathological review. The patients' age ranged from 1 month to 59 years; there were 46 males and 20 females. None of the patients had clinical signs of NBCCS or had first degree relatives with NBCCS. All tumors were diagnosed according to the revised WHO classification of brain tumors using standard histological methods including HE and reticulin stains and immunohistochemical reactions (Kleihues *et al.* (1993) *Histological typing of tumours of the central nervous system,* Springer Verlag, New York). Differentiation was assessed by immunostaining for embryonal neural cell adhesion molecule (NCAM), neuron-specific enolase, synaptophysin and glial acidic fibrillary protein. Frozen tumour samples were obtained at the time of surgical resection, snap frozen in liquid nitrogen and stored at -80°C.

### DNA extraction, LOH analysis.

Tumour fragments were selected for extraction of DNA after careful examination of corresponding frozen sections to exclude contaminating necrotic debris or normal cerebellar tissue and to determine the histological characteristics of the tumors. DNA was extracted by standard proteinase K digestion and phenol/chloroform extraction (Albrecht *et al*, 1994). Loss of heterozygosity was determined by microsatellite analysis with the markers D9S287 and D9S 197 which were tightly linked to the *PTC* gene and with two additional markers on 9q (D9S302, D9S303) essentially as previously described (Albrecht *et al.* (1994) *Neuropathol. Appl. Neurobiol.* 20:74-81; Kraus *et al.* (1996) *Int. J. Cancer* 67: 11-15).

### SSCP analysis and DNA sequencing

SSCP analysis of exons 2-22 was performed using 22 primer pairs (previous Examples and Hakin *et al*, 1996). PCR containing 50 mM KCl, 1.0-2.5 mM MgCl₂, 10 mM Tris-HCl (pH 8.5), 0.01% w/v gelatin and 200 mM of each dNTP, 2 µM of the primers and 0.25 units Taq polymerase (Gibco-BRL) on a Uno Thermoblock^{™} cycler (Biometra). The products were analyzed on polyacrylamide gels with different acrylamide concentrations and acrylamide/bisacrylamide ratios. Gel composition and electrophoresis conditions were optimized for each individual primer pair. The single and double strands were visualized by silver staining as previously described (Albrecht *et al*, 1994). PCR products which showed a gel mobility shift were excised from the wet gel, eluted (Koch *et al*, 1996) and reamplified by PCR with the same primers. The resulting products were purified using spin columns (Qiagen^{™} quick spin), and 20 ng used to cycle sequencing with a fluorescent dideoxy terminator kit (ABI). The products were analyzed on an Applied Biosystems model 373A DNA sequencer.

### Isolation of RNA, quantitative RT-PCR for PTCH mRNA.

Total cellular RNA was extracted by lysis in guanidinium isothiocyanate and ultracentrifugation through a cesium chloride cushion (Koch *et al.,* 1996) or by extraction with the Trizol^{™} reagent (Gibco-BRL) following the manufacturer's instructions. Again, individual samples were preexamined by frozen section histology to document the histopathological appearance of the specimen. Contaminating residual genomic DNA was removed by digestion with RNAse free DNAse (Boehringer). RNA standards with internal deletions for human *PTC* and the housekeeping genes *β₂-microglobulin* and GAPDH were generated by *in vitro* mutagenesis and *in vitro* transcription (Horton and Pease (1991) in *Direct Mutagenesis-A Practical Approach,* McPerson, ed., pp.217-247 (IRL Press, Oxford). In order to achieve a semi-quantitative assessment, pre-evaluated amounts of the specific standards RNAs covering the equimolar range of the corresponding mRNA transcripts were added to the MB sample RNAs which were then reverse transcribed using the SuperScript^{™} Preamplification System (Gibco-BRL) with random hexamers as primers in a final volume of 10 µl. 0.5 µl of the cDNA was used as a template in RT-PCR reactions for amplification of *PTCH,* and the housekeeping genes. The primers used were: *PTCH,* 5'ACATGTACAACAGGCAGTGG-3 [SEQ ID NO:61] and 5'-GCAAGGAGGTTTACCTAGG-3' [SEQ ID NO.62], product size, wild type 192bp, standard 182 bp; GAPDH, 5'-TGCCAAGGCTGTGGGCAAGG-3' [SEQ ID NO:63] and 5'-GCTTCACCACCTTCTTGATG-3' [SEQ ID NO:64] product size, wild type 152bp, standard 142bp; β*₂-microglobulin*, 5'-GCTGTGACAAAGTCACATGG-3' [SEQ ID NO:65] and 5'-GATGCTGCTTACATGTCTCG-3' [SEQ ID NO:66], product size, wild type 148bp, standard 130bp. One of the primers for each gene was labeled with a fluorescent dye. All primers were chosen from adjacent exons spanning intronic sequences in order to avoid signals of the cDNA product size caused by residual genomic DNA. The PCR products were separated and analyzed on an Applied Biosystems model 373A DNA sequencer using the Genescan software (ABI). The expression levels of the individual genes were calculated from the signal ratios of the samples to the standards. The relative expression of *PTCH* mRNA to the housekeeping genes was defined as the ratio of the respective expression levels (Figure 10).

The human *PTCH* gene spans 34 kB and has at least 23 exons. SSCP screening of DNA samples from 68 sporadic MBs revealed band shifts in 6 samples (Table 7). Three of these were identified as silent polymorphisms. In three other tumors, the variants were not found in the corresponding germline DNA or in normal control DNA samples. Two mutations in exons 6 and 10, respectively, resulted in a frame shift with premature truncation of the protein (Table 7 and Figure 9). The third mutation (D86) was a six base pair in frame deletion in exon 10 leading to the deletion of two amino acids in transmembrane region 3. This deletion may cause significant structural alterations of the PTCH protein and may result in loss of function. This was the case in tumours D86 and D322 which showed LOH as well as mutated *PTC* allele. In case D292 without detectable LOH at 9q, only a single SSCP band shift was found (in exon 10). Mutations of the other allele may be present but may not have been detected by SSCP screening because of the limited sensitivity of SSCP. Only the coding exons were screened so that mutations in other regions such as regulatory domains would not have been identified with this approach. A systematic sequencing analysis may uncover additional *PTCH* mutations in Mbs.

In this Example, mutations were detected in a distinct histopathological variant or medulloblastomas (MB), the so-called modular or "desmoplastic" MB. According to the WHO classification this variant is characterized by islands of lower cellularity surrounded by densely packed, highly proliferative cells which produce a dense intercellular reticulin fiber network. The more frequent "classical" MB lacks this nodular appearance and reticulin pattern.

**TABLE 7**

| **MUTATIONAL ANALYSIS of the *PTCH* GENE IN MEDULLOBLASTOMAS** | | | | | | |
|---|---|---|---|---|---|---|
| **a, Mutations** | | | | | | |
| Tumour | MB variant | Age/sex | LOH on 9q | Exon | Nucleotide change | Protein change |
| D 86 | desmoplastic | 4y, male | yes | 10 | 1444de16 | del Gly-Leu |
| D 292 | desmoplastic | ly, female | no | 10 | 1393insTGCC truncation | frameshift, |
| D 322 | desmoplastic | 51y, male | yes | 6 | 887delG truncation | frameshift, |
| | | | | | | |

| **b, Polymorphisms** | | | | | | |
|---|---|---|---|---|---|---|
| Tumour | MB variant | Age/sex | LOH on 9q | Exon | Nucleotide change | Protein change |
| D 230 11 | classical | 13y, female | no | 13 | C2037T | no |
| D 338 | classical | 13y, male | n.a. | 2 | C306T | no |
| D 358 | classical | 10y, female | n.a. | 2 | C306T | no |

**TABLE 8**

| **EXPRESSION OF THE *PTCH* GENE IN MEDULLOBLASTOMAS** | | | | | |
|---|---|---|---|---|---|
| Sample | MB subtype | LOH on 9q | *PTCH* mutation detected by SSCP | mRNA expression ratio *PTCH*/*GAPDH* | mRNA expression ratio *PTCH*/*β2-*microglobulin |
| D 338 | classical | n.a.* | no | 5.8(3.9-7.4)** | 10.3 (8.2-14.2) |
| D 230 II | classical | no | no | 1.7 | n.a. |
| D 286 | classical | n.a. | no | 1.2 | n.a. |
| D 245 II | classical | no | no | 0.7 | n.a. |
| D 446 | classical | no | no | 1.8 (0.8-2.8) | 1.5(1.0-1.8) |
| D 447 | classical | no | no | 0.6 | n.a. |
| | | | | | |
| D 86 | desmopl. | yes | yes, exon 10 | 3.6 (1.4-5.4) | 2.6 (1.7-3.9) |
| D 292 | desmopl. | no | yes, exon 10 | 0.6 (0.6-0.6) | 0.3 (0.3-0.4) |
| D 322 | desmopl. | yes | yes, exon 6 | 1.1 | n.a. |
| D 448 | desmopl. | yes | no | 4.3 (3.3-5.5) | 2.9 (2.5-3-6) |
| D 398 | desmopl. | no | no | 26.5(22.4-30.0) | 18.4 (12.0-24.4) |
| D 444 | desmopl. | yes | no | 4.1 (3.7-4.7) | 4.5 (3.3-5.5) |
| D 365*** | desmopl. | n.a. | no | 0.3 (0.2-0.4) | 0.1 (0.1-0.2) |
| | | | | | |
| Cerebellum | - | n.a. | n.a. | 2.5 (1.55-3.64) | 1.76 (1.42-2.09) |

| | | | | | |
|---|---|---|---|---|---|
| *n.a., not analyzed; ** mean and range of relative expression (analyzed by semi-quantitative RT-PCR); ***D365, cell line Daoy. | | | | | |

### Example 6

### Most Germ Line Mutations in the NBCCS Gene lead to a Premature Termination of the PATCHED Protein

In this Example, the inventors screened DNA samples form 71 unrelated NBCCS individuals for mutations in the *PTCH* exons using single strand conformational polymorphism (SSCP) analysis. In total, 28 mutations were identified and characterised by direct sequencing of PCR products. The majority of these mutations (86%) lead to premature truncation of the PTCH protein. Analysis of phenotype in individuals with truncating mutations revealed no statistically significant correlation between genotype and phenotype in NBCCS.

### Materials and Methods

### Subjects and samples

The patients, most of whom were from Australia and New Zealand, were diagnosed according to the clinical criteria in Shanley *et al.* (1994) *supra.* Of the 71 NBCCS patients analyzed 25 show clear familial presentation and 46 are apparently sporadic.

### SSCP analysis

A combined SSCP and heteroduplex analysis was performed as previously described (Hahn *et al*. (1996) *supra*). DNA from 71 unrelated NBCCS individuals was amplified with primers to all but exons 1b (alternative first exon homologous to murine exon 1), 12 and 20 (for which acceptable primers are not yet available) of the human *PTCH* gene. Primer sequences and conditions were as hereinbefore described as well as (Hahn *et al*. (1996) *supra*).

### Sequencing

DNA from samples showing SSCP variants was reamplified and purified for automated sequencing using PCR Spinclean^{™} Columns (Progen Industries). DNA concentration was ascertained by agarose gel electrophoresis and 25-35 ng of product was used for each automated sequencing run. Cycle sequencing was performed using Amplitaq FS polymerase and dye labelled terminator chemistry (Perkin Elmer Cetus), and samples were analyzed on an PEC 373A electrophoresis apparatus. Where mutations were confirmed by manual sequencing the purified products were ligated into GEM-T vector (Promega) and the resulting clones were sequenced with a T7 Sequencing Kit (Pharmacia).

### Southern analysis

Southern blots were made as previously described (Chenevix-Trench *et al*. 1992), using *Eco*R1 and *Hin*dIII restriction enzymes and hybridized with *PTCH* cDNA probes 13B and 16C.

### Statistical analysis

Linear regression analysis was used to examine genotype-phenotype associations.

### Results

### Identification of PTCH Mutations

DNA from 71 individuals with NBCCS was screened for mutations in the *PTC* gene by a combined SSCP/heteroduplex analysis. Based upon sequencing of samples showing SSCP variation, 28 putative disease-associated mutations have been fully characterised (Table 9). While one mutation, a CT deletion at nucleotide position 244, was seen in 3 apparently unrelated individuals, all other mutations were only detected in a single NBCCS family. No clustering of mutations has been observed, with mutations identified in most exons and in positions corresponding to all of the major domain types of the PTC protein (Fig. 11).

The majority (24/28; 86%) of mutations detected are predicted to result in truncation of the PTCH protein either by introduction of a stop codon or by frameshift due to insertion or deletion (Table 9). Two of the mutations were predicted to be splice variants based on the fact that one altered a consensus 3' splice site, while the other involved an insertion of 21bp in intron 10, 8bp upstream of the start of exon 11. This second mutation was presumed to cause aberrant splicing based on the fact that it moves the branch site from position 27 to 48bp upstream of the 3' splice site. This 7bp consensus sequence is generally located approximately 18 to 37bp upstream of the splice site, and its location is considered to be important in the splicing reaction. The remaining two mutations are missense mutations which both alter residues within transmembrane domains of the PTCH protein. One (PP) is a transversion of GAT to TAT at nucleotide 1525, substituting a tyrosine for an aspartate in the fourth transmembrane domain; the other (RS) is a transversion of a GGC to CGC at nucleotide 3193, substituting an arginine for a glycine in the ninth transmembrane domain of the PTCH protein.

In addition to disease-associated mutations, several variants were designated polymorphisms based on their presence in unaffected individuals, or the finding that the underlying sequence changes did not alter the encoded amino acids. For samples in which no SSCP variation was found, the sequence of each exon of the *PTC* gene is currently being determined. DNA from 38 patients in which a mutation was not found by SSCP was also examined by Southern analysis using probes which span the gene. Variants were detected in two patients. In each case, a single additional band (3 and 3.75 kb respectively) was seen on *Hind*III blots. No variants were present in these individuals on *EcoRI, BamHI and Pst I* digests, therefore, those seen on *Hind*III blots are unlikely to represent gross rearrangements. The probability of at least one of these variants being a disease-related point mutation is increased by its segregation with disease in a family. No family members were available for study in the case of the second variant. The underlying mutations in these individuals are yet to be determined.

### Analysis of genotype-phenotype associations

Although most of the mutations found to date are predicted to truncate the protein, it remains to be determined whether all ablate its function. In order to address this, preliminary analysis of genotype-phenotype associations in this complex syndrome was performed, based on the 24 families with protein-truncating mutations. Several aspects of the NBCCS phenotype were used as approximate parameters of disease severity. The inventors examined the number of major features (BCCs, jaw cysts, pitting and falcine calcification) seen in individuals at the time of diagnosis, the age at which the individual manifested BCCs and the age at which jaw cysts were detected. Individuals under the age of 20 years were not included in this analysis due to the age-dependent expression of these features. Similarly, analysis of age of BCC onset was restricted to Australasian patients to limit the influence of ultraviolet exposure in promoting BCC development. When a mutation was known to be present in a number of individuals within a family, phenotypic data were averaged across all relevant family members. No correlations between the age of onset of BCCs (R²=0.001) or jaw cysts (R²=0.023), or the number of major features (R²=0.015), and nucleotide position of the mutation, were found, indicating that for these features at least, there is no clear correlation between phenotype and location of the truncating mutation. Although it was not appropriate to use statistical analyses to compare truncating mutations with missense and splice variants due to the small number of the latter type of mutations, the individuals the inventors have analyzed with missense and splice mutations show a classic NBCCS phenotype and would not be classed as mildly affected.

The phenotypes of individuals in the three families which share a common mutation (244delCT) were evaluated, and shown to vary considerably. All five affected members of family CB have a cleft or very high arched palate but this was not observed in the HC or BK families, both of whom show the typical range of NBCCS features. This suggests that the molecular nature of the *PTC* mutation is not entirely responsible for the phenotype in NBCCS. Interestingly, BK carries a new mutation of *PTC* so this mutation must have arisen at least twice.

The inventors have identified mutations in the *PTC* gene in 28 unrelated individuals with NBCCS and found no evidence of any association between genotype and phenotype. In 24 families with protein-truncating mutations, no significant correlation between phenotype and location of the truncating mutation was found. This differs from breast and ovarian cancer where 3' mutations in the BRCA1 gene are less likely to predispose to ovarian cancer than are 5' mutations presumably because of residual activity of proteins resulting from 3' mutations.

**TABLE 9**

| **GERM-LINE MUTATIONS IN THE *PTC* GENE IN NBCCS INDIVIDUALS** | | | |
|---|---|---|---|
| | EXON | MUTATION^{b} | EFFECT ON CODING^{c} |
| PATIENT^{a} | Missense | | |
| PP (S) | 11 | G1525T | D-Y at 513 |
| | | | |
| RS (S) | 18 | G3193C | G-R at 1069 |
| JHG(S) | 3 | C391T | R-X at 135 |
| JM (F) | 8 | G1148A^{d} | W-X at 387 |
| TM (S) | 10 | G1368A | W-X at 460 |
| DD (S) | 13 | C2050T | Q-X at 688 |
| BH (S) | 13 | C2068T | Q-X at 694 |
| PB (F) | 17 | C3015A | Y-X at 1009 |

| Insertions, Deletions, and Duplications | | | |
|---|---|---|---|
| HC, CB(F); BK(S)^{c} | 2 | 244delCT | Frameshift |
| JHK (S) | 2 | 271insA | Frameshift |
| GS (S) | 3 | 464insAC | Frameshift |
| MC (F) | 6 | 804del37^{d} | Frameshift |
| JRN (S) | 6 | 929delC | Frameshift |
| DS (F) | 10 | 1370de176 | Frameshift |
| CM (S) | 11 | 1497dup8 | Frameshift |
| MP (F) | 13 | 2183delTC | Frameshift |
| TH (F) | 14 | 2320insA | Frameshift |
| LK (S) | 14 | 2392delA | Frameshift |
| DC (S) | 15 | 2574delA | Frameshift |
| KS (S)^{c} | 15 | 2583delC^{d} | Frameshift |
| WS (F) | 15 | 2596complex^{f} | Frameshift |
| DE (F) | 16 | 2748insC | Frameshift |
| JRD (F) | 16 | 2749dup7 | Frameshift |
| JW (S) | 19 | 3352delAT | Frameshift |

| Splicing | | | |
|---|---|---|---|
| AE (F) | Intron 7 | A1055-2C | 3' splice site |
| IMc (S) | Intron 10 | 1493-8ins21 | Putative splice variant |

| Polymorphisms^{g} | | | |
|---|---|---|---|
| | 2 | A or T at 312 | No change I108 |
| | 3 | T or C at 417 | No change T143 |
| | 4 | A or G at 588 | No change E200 |
| | 5 | A or G at 723 | No change T245 |
| | 7 | T or C at 1023 | No change G345 |
| | Intron 10 | G or C at 1493-39 | |
| | Intron 11 | A or T at 1591+29 | |
| | Intron 18 | delTT at 3294+27 | |
| | 22 | T or C at 3933 | No change L1315 |

| | | | |
|---|---|---|---|
| ^{a} S = sporadic mutation; F = familial mutation. ^{b} As per Genbank entry U43148. ^{c} As per Genbank entry U59464. ^{d} As previously reported b Hahn *et al*., 1996. ^{e} Sporadic cases in which parents were analyzed and no mutation was seen. ^{f} Complex mechanism involving insertion and deletion. ^{g} Polymorphisms in exons 3, 4, and 7 are rare. | | | |

Sequence ID NO: 1: *NBCCS (PTC)* cDNA
   (2) INFORMATION FOR SEQ ID NO:1:
      (i) SEQUENCE CHARACTERISTICS:
         (A) LENGTH: 6568 base pairs
         (B) TYPE: nucleic acid
         (C) STRANDEDNESS: single
         (D) TOPOLOGY: linear
      (ii) MOLECULE TYPE: cDNA
      (ix) FEATURE:
         (A) NAME/KEY: CDS
         (B) LOCATION: 442..4329
      (xi) SEQUENCE DESCRIPTION: SEQ ID NO:1:
SEQ ID NO: 60: Amino acid sequence of human *NBCCS* (*PTC*).
   (2) INFORMATION FOR SEQ ID NO:60:
      (i) SEQUENCE CHARACTERISTICS:
         (A) LENGTH: 1296 amino acids
         (B) TYPE: amino acid
         (D) TOPOLOGY: linear
      (ii) MOLECULE TYPE: protein
      (xi) SEQUENCE DESCRIPTION: SEQ ID NO:60:
   Met Phe Asn Pro Gln Leu Met Ile Gln Thr Pro Lys Glu Glu Gly Ala
   1               5                   10                  15
   Asn Val Leu Thr Thr Glu Ala Leu Leu Gln His Leu Asp Ser Ala Leu
               20                  25                  30
   Gln Ala Ser Arg Val His Val Tyr Met Tyr Asn Arg Gln Trp Lys Leu
           35                  40                  45
   Glu His Leu Cys Tyr Lys Ser Gly Glu Leu Ile Thr Glu Thr Gly Tyr
       50                  55                  60
   Met Asp Gln Ile Ile Glu Tyr Leu Tyr Pro Cys Leu Ile Ile Thr Pro
   65                  70                  75                  80
   Leu Asp Cys Phe Trp Glu Gly Ala Lys Leu Gln Ser Gly Thr Ala Tyr
                   85                  90                  95
   Leu Leu Gly Lys Pro Pro Leu Arg Trp Thr Asn Phe Asp Pro Leu Glu
               100                 105                 110
   Phe Leu Glu Glu Leu Lys Lys Ile Asn Tyr Gln Val Asp Ser Trp Glu
           115                 120                 125
   Glu Met Leu Asn Lys Ala Glu Val Gly His Gly Tyr Met Asp Arg Pro
       130                 135                 140
   Cys Leu Asn Pro Ala Asp Pro Asp Cys Pro Ala Thr Ala Pro Asn Lys
   145                 150                 155                 160
   Asn Ser Thr Lys Pro Leu Asp Met Ala Leu Val Leu Asn Gly Gly Cys
                   165                 170                 175
   His Gly Leu Ser Arg Lys Tyr Met His Trp Gln Glu Glu Leu Ile Val
               180                 185                 190
   Gly Gly Thr Val Lys Asn Ser Thr Gly Lys Leu Val Ser Ala His Ala
           195                 200                 205
   Leu Gln Thr Met Phe Gln Leu Met Thr Pro Lys Gln Met Tyr Glu His
       210                 215                 220
   Phe Lys Gly Tyr Glu Tyr Val Ser His Ile Asn Trp Asn Glu Asp Lys
   225                 230                 235                 240
   Ala Ala Ala Ile Leu Glu Ala Trp Gln Arg Thr Tyr Val Glu Val Val
                   245                 250                 255
   His Gln Ser Val Ala Gln Asn Ser Thr Gln Lys Val Leu Ser Phe Thr
               260                 265                 270
   Thr Thr Thr Leu Asp Asp Ile Leu Lys Ser Phe Ser Asp Val Ser Val
           275                 280                 285
   Ile Arg Val Ala Ser Gly Tyr Leu Leu Met Leu Ala Tyr Ala Cys Leu
       290                 295                 300
   Thr Met Leu Arg Trp Asp Cys Ser Lys Ser Gln Gly Ala Val Gly Leu
   305                 310                 315                 320
   Ala Gly Val Leu Leu Val Ala Leu Ser Val Ala Ala Gly Leu Gly Leu
                   325                 330                 335
   Cys Ser Leu Ile Gly Ile Ser Phe Asn Ala Ala Thr Thr Gln Val Leu
               340                 345                 350
   Pro Phe Leu Ala Leu Gly Val Gly Val Asp Asp Val Phe Leu Leu Ala
           355                 360                 365
   His Ala Phe Ser Glu Thr Gly Gln Asn Lys Arg Ile Pro Phe Glu Asp
       370                 375                 380
   Arg Thr Gly Glu Cys Leu Lys Arg Thr Gly Ala Ser Val Ala Leu Thr
   385                 390                 395                 400
   Ser Ile Ser Asn Val Thr Ala Phe Phe Met Ala Ala Leu Ile Pro Ile
                   405                 410                 415
   Pro Ala Leu Arg Ala Phe Ser Leu Gln Ala Ala Val Val Val Val Phe
               420                 425                 430
   Asn Phe Ala Met Val Leu Leu Ile Phe Pro Ala Ile Leu Ser Met Asp
           435                 440                 445
   Leu Tyr Arg Arg Glu Asp Arg Arg Leu Asp Ile Phe Cys Cys Phe Thr
       450                 455                 460
   Ser Pro Cys Val Ser Arg Val Ile Gln Val Glu Pro Gln Ala Tyr Thr
   465                 470                 475                 480
   Asp Thr His Asp Asn Thr Arg Tyr Ser Pro Pro Pro Pro Tyr Ser Ser
                   485                 490                 495
   His Ser Phe Ala His Glu Thr Gln Ile Thr Met Gln Ser Thr Val Gln
               500                 505                 510
   Leu Arg Thr Glu Tyr Asp Pro His Thr His Val Tyr Tyr Thr Thr Ala
           515                 520                 525
   Glu Pro Arg Ser Glu Ile Ser Val Gln Pro Val Thr Val Thr Gln Asp
       530                 535                 540
   Thr Leu Ser Cys Gln Ser Pro Glu Ser Thr Ser Ser Thr Arg Asp Leu
   545                 550                 555                 560
   Leu Ser Gln Phe Ser Asp Ser Ser Leu His Cys Leu Glu Pro Pro Cys
                   565                 570                 575
   Thr Lys Trp Thr Leu Ser Ser Phe Ala Glu Lys His Tyr Ala Pro Phe
               580                 585                 590
   Leu Leu Lys Pro Lys Ala Lys Val Val Val Ile Phe Leu Phe Leu Gly
           595                 600                 605
   Leu Leu Gly Val Ser Leu Tyr Gly Thr Thr Arg Val Arg Asp Gly Leu
       610                 615                 620
   Asp Leu Thr Asp Ile Val Pro Arg Glu Thr Arg Glu Tyr Asp Phe Ile
   625                 630                 635                 640
   Ala Ala Gln Phe Lys Tyr Phe Ser Phe Tyr Asn Met Tyr Ile Val Thr
                   645                 650                 655
   Gln Lys Ala Asp Tyr Pro Asn Ile Gln His Leu Leu Tyr Asp Leu His
               660                 665                 670
   Arg Ser Phe Ser Asn Val Lys Tyr Val Met Leu Glu Glu Asn Lys Gln
           675                 680                 685
   Leu Pro Lys Met Trp Leu His Tyr Phe Arg Asp Trp Leu Gln Gly Leu
       690                 695                 700
   Gln Asp Ala Phe Asp Ser Asp Trp Glu Thr Gly Lys Ile Met Pro Asn
   705                 710                 715                 720
   Asn Tyr Lys Asn Gly Ser Asp Asp Gly Val Leu Ala Tyr Lys Leu Leu
                   725                 730                 735
   Val Gln Thr Gly Ser Arg Asp Lys Pro Ile Asp Ile Ser Gln Leu Thr
               740                 745                 750
   Lys Gln Arg Leu Val Asp Ala Asp Gly Ile Ile Asn Pro Ser Ala Phe
           755                 760                 765
   Tyr Ile Tyr Leu Thr Ala Trp Val Ser Asn Asp Pro Val Ala Tyr Ala
       770                 775                 780
   Ala Ser Gln Ala Asn Ile Arg Pro His Arg Pro Glu Trp Val His Asp
   785                 790                 795                 800
   Lys Ala Asp Tyr Met Pro Glu Thr Arg Leu Arg Ile Pro Ala Ala Glu
                   805                 810                 815
   Pro Ile Glu Tyr Ala Gln Phe Pro Phe Tyr Leu Asn Gly Leu Arg Asp
               820                 825                 830
   Thr Ser Asp Phe Val Glu Ala Ile Glu Lys Val Arg Thr Ile Cys Ser
           835                 840                 845
   Asn Tyr Thr Ser Leu Gly Leu Ser Ser Tyr Pro Asn Gly Tyr Pro Phe
       850                 855                 860
   Leu Phe Trp Glu Gln Tyr Ile Gly Leu Arg His Trp Leu Leu Leu Phe
   865                 870                 875                 880
   Ile Ser Val Val Leu Ala Cys Thr Phe Leu Val Cys Ala Val Phe Leu
                   885                 890                 895
   Leu Asn Pro Trp Thr Ala Gly Ile Ile Val Met Val Leu Ala Leu Met
               900                 905                 910
   Thr Val Glu Leu Phe Gly Met Met Gly Leu Ile Gly Ile Lys Leu Ser
           915                 920                 925
   Ala Val Pro Val Val Ile Leu Ile Ala Ser Val Gly Ile Gly Val Glu
       930                 935                 940
   Phe Thr Val His Val Ala Leu Ala Phe Leu Thr Ala Ile Ser Asp Lys
   945                 950                 955                 960
   Asn Arg Arg Ala Val Leu Ala Leu Glu His Met Phe Ala Pro Val Leu
   965 970 975
   Asp Gly Ala Val Ser Thr Leu Leu Gly Val Leu Met Leu Ala Gly Ser
   980 985 990
   Glu Phe Asp Phe Ile Val Arg Tyr Phe Phe Ala Val Leu Ala Ile Leu
   995 1000 1005
   Thr Ile Leu Gly Val Leu Asn Gly Leu Val Leu Leu Pro Val Leu Leu
   1010 1015 1020
   Ser Phe Phe Gly Pro Tyr Pro Glu Val Ser Pro Ala Asn Gly Leu Asn
   1025 1030 1035 1040
   Arg Leu Pro Thr Pro Ser Pro Glu Pro Pro Pro Ser Val Val Arg Phe
   1045 1050 1055
   Ala Met Pro Pro Gly His Thr His Ser Gly Ser Asp Ser Ser Asp Ser
   1060 1065 1070
   Glu Tyr Ser Ser Gln Thr Thr Val Ser Gly Leu Ser Glu Glu Leu Arg
   1075 1080 1085
   His Tyr Glu Ala Gln Gln Gly Ala Gly Gly Pro Ala His Gln Val Ile
   1090 1095 1100
   Val Glu Ala Thr Glu Asn Pro Val Phe Ala His Ser Thr Val Val His
   1105 1110 1115 1120
   Pro Glu Ser Arg His His Pro Pro Ser Asn Pro Lys Gln Gln Pro His
   1125 1130 1135
   Leu Asp Ser Gly Ser Leu Pro Pro Gly Arg Gln Gly Gln Gln Pro Arg
   1140 1145 1150
   Arg Asp Pro Pro Arg Lys Gly Leu Trp Pro Pro Leu Tyr Arg Pro Arg
   1155 1160 1165
   Arg Asp Ala Phe Glu Ile Ser Thr Glu Gly His Ser Gly Pro Ser Asn
   1170 1175 1180
   Arg Ala Arg Trp Gly Pro Arg Gly Ala Arg Ser His Asn Pro Arg Asn
   1185 1190 1195 1200
   Pro Thr Ser Thr Ala Met Gly Ser Ser Val Pro Gly Tyr Cys Gln Pro
   1205 1210 1215
   Ile Thr Thr Val Thr Ala Ser Ala Ser Val Thr Val Ala Val His Pro
   1220 1225 1230
   Pro Pro Val Pro Gly Pro Gly Arg Asn Pro Arg Gly Gly Leu Cys Pro
   1235 1240 1245
   Gly Tyr Pro Glu Thr Asp His Gly Leu Phe Glu Asp Pro His Val Pro
   1250 1255 1260
   Phe His Val Arg Cys Glu Arg Arg Asp Ser Lys Val Glu Val Ile Glu
   1265 1270 1275 1280
   Leu Gln Asp Val Glu Cys Glu Glu Arg Pro Arg Gly Ser Ser Ser Asn
   1285 1290 1295

## Claims

1. An isolated NBCCS polypeptide encoded by a tumor suppressor gene associated with nevoid basal cell carcinoma syndrome (NBCCS) and various cancers including various sporadic basal cell carcinomas (BCCs), wherein the polypeptide has the amino acid sequence of SEQ ID NO:60.

2. An isolated nucleic acid encoding a tumor suppressor gene associated with nevoid basal cell carcinoma syndrome (NBCCS) and various cancers including various sporadic basal cell carcinomas (BCCs), wherein the nucleic acid encodes a polypeptide that has the amino acid sequence of SEQ ID NO:60.

3. The nucleic acid of claim 2, wherein the nucleic acid has the sequence set forth at nucleotides 442 to 4329 of SEQ ID: NO:1.

4. A recombinant vector comprising a nucleic acid of one of claims 2 or 3.

5. A host cell expressing the nucleic acids of one of claims 2 or 3 or the vector of claim 4.

6. A composition for the treatment of nevoid basal cell carcinoma syndrome (NBCCS) and various cancers including various sporadic basal cell carcinomas (BCCs) in a mammal, wherein the composition comprises:
(a1) the polypeptide of claim 1; or
(a2) the nucleic acid of one of claims 2 or 3; or
(a3) the vector of claim 4; and
(b) a pharmaceutically acceptable carrier.

7. The composition of claim 6, wherein said pharmaceutically acceptable carrier is suitable for topical or for parenteral administration to the mammal.

8. The composition of one of claims 6 or 7, wherein the mammal is human.

9. Use of an isolated polypeptide encoded by a tumor suppressor gene associated with nevoid basal cell carcinoma syndrome (NBCCS) and various cancers including various sporadic basal cell carcinomas (BCCs) for the manufacture of a medicament for mitigating a symptom of nevoid basal cell carcinoma syndrome, or a basal cell carcinoma or of solar keratoses in a mammal, wherein the polypeptide has the amino acid sequence of SEQ ID NO:60.

10. Use of an isolated nucleic acid encoding a tumor suppressor gene associated with nevoid basal cell carcinoma syndrome (NBCCS) and various cancers including various sporadic basal cell carcinomas (BCCs) for the manufacture of a medicament for mitigating a symptom of nevoid basal cell carcinoma syndrome, or a basal cell carcinoma or of solar keratoses in a mammal, wherein the nucleic acid comprises a coding region encoding a polypeptide with of SEQ ID NO:60.

11. The use of claim 10, wherein said nucleic acid has the sequence of nucleotides 442 to 4329 of SEQ ID NO:1.

12. The use according to any one of claims 9 to 11, wherein the mammal is human.

## Patentansprüche

1. Isoliertes NBCCS Polypeptid, codiert von einem Tumorsuppressorgen, das mit dem Nävusbasalzellkarzinomsyndrom (NBCCS) und verschiedenen Krebsarten, einschliesslich verschiedener sporadischer Basalzellkarzinome (BCCs), assoziiert ist, wobei das Polypeptid die Aminosäuresequenz SEQ ID NO:60 aufweist.

2. Isolierte Nukleinsäure, die ein Tumorsuppressorgen codiert, das mit dem Nävusbasalzellkarzinomsyndrom (NBCCS) und verschiedenen Krebsarten, einschliesslich verschiedener sporadischer Basalzellkarzinome (BCCs), assoziiert ist, wobei die Nukleinsäure ein Polypeptid codiert, das die Aminosäuresequenz von SEQ ID NO:60 aufweist.

3. Nukleinsäure nach Anspruch 2, wobei die Nukleinsäure die bei den Nukleotiden 442 bis 4329 der SEQ ID NO:1 dargelegte Sequenz aufweist.

4. Rekombinanter Vektor, eine Nukleinsäure nach einem der Ansprüche 2 oder 3 umfassend.

5. Wirtszelle, welche die Nukleinsäuren eines der Ansprüche 2 oder 3 oder den Vektor nach Anspruch 4 exprimiert.

6. Zusammensetzung zur Behandlung eines Nävusbasalzellkarzinomsyndroms (NBCCS) und verschiedener Krebsarten, einschliesslich verschiedener sporadischer Basalzellkarzinome (BCCs), in einem Säugetier, wobei die Zusammensetzung umfasst:
(a1) das Polypeptid nach Anspruch 1; oder
(a2) die Nukleinsäure nach einem der Ansprüche 2 oder 3; oder
(a3) den Vektor nach Anspruch 4; und
(b) einen pharmazeutisch akzeptablen Trägerstoff.

7. Zusammensetzung nach Anspruch 6, wobei der pharmazeutisch akzeptable Trägerstoff für die topische oder parenterale Verabreichung an das Säugetier geeignet ist.

8. Zusammensetzung nach einem der Ansprüche 6 oder 7, wobei das Säugetier ein Mensch ist.

9. Verwendung eines isolierten Polypeptids, das von einem Tumorsuppressorgen codiert ist, das mit dem Nävusbasalzellkarzinomsyndrom (NBCCS) und verschiedenen Krebsarten, einschliesslich verschiedener sporadischer Basalzellkarzinome (BCCs), assoziiert ist, für die Herstellung eines Medikaments zur Milderung eines Symptoms des Nävusbasalzellkarzinomsyndroms, oder eines Basalzellkarzinoms oder einer durch Sonneneinstrahlung bewirkten Keratosis in einem Säugetier, wobei das Polypeptid die Aminosäuresequenz SEQ ID NO:60 aufweist.

10. Verwendung einer isolierten Nukleinsäure, die ein Tumorsuppressorgen codiert, das mit dem Nävusbasalzellkarzinomsyndrom (NBCCS) und verschiedenen Krebsarten, einschliesslich sporadischer Basalzellkarzinome (BCCs), assoziiert ist, für die Herstellung eines Medikaments zur Milderung eines Symptoms des Nävusbasalzellkarzinomsyndroms oder eines Basalzellkarzinoms oder einer durch Sonneneinstrahlung bewirkten Keratosis in einem Säugetier, wobei die Nukleinsäure einen Codierungsbereich umfasst, der ein Polypeptid mit SEQ ID NO:60 codiert.

11. Verwendung nach Anspruch 10, wobei die Nukleinsäure die Sequenz der Nukleotide 442 bis 4329 der SEQ ID NO:1 aufweist.

12. Verwendung nach einem der Ansprüche 9 bis 11, wobei das Säugetier ein Mensch ist.

## Revendications

1. Polypeptide NBCCS isolé codé par un gène suppresseur de tumeur associé au syndrome de carcinome basocellulaire naevoïde (NBCCS) et à différents cancers, notamment à divers carcinomes basocellulaires sporadiques (BCCs), tel que le polypeptide possède la séquence d'acides aminés selon la SEQ ID N° 60.

2. Acide nucléique isolé codant pour un gène suppresseur de tumeur associé au syndrome de carcinome basocellulaire naevoïde (NBCCS) et à différents cancers, notamment divers carcinomes basocellulaires sporadiques (BCCs), tel que l'acide nucléique code pour un polypeptide ayant la séquence d'acides aminés selon la SEQ ID N° 60.

3. Acide nucléique selon la revendication 2, tel que l'acide nucléique a la séquence exposée dans les nucléotides 442 à 4329 de la séquence SEQ ID N° 1.

4. Vecteur de recombinaison contenant un acide nucléique selon l'une des revendications 2 ou 3.

5. Cellule hôte exprimant les acides nucléiques selon l'une des revendications 2 ou 3 ou le vecteur selon la revendication 4.

6. Composition pour le traitement du syndrome de carcinome basocellulaire naevoïde (NBCCS) et de différents cancers, notamment divers carcinomes basocellulaires sporadiques (BCCs) chez un mammifère, laquelle composition contient :
(a1) le polypeptide selon la revendication 1 ; ou
(a2) l'acide nucléique selon l'une des revendications 2 ou 3 ; ou
(a3) le vecteur selon la revendication 4 ; et
(b) un véhicule pharmaceutiquement acceptable.

7. Composition selon la revendication 6, telle que ledit véhicule pharmaceutiquement acceptable permet une administration topique ou parentérale au mammifère.

8. Composition selon l'une des revendications 6 ou 7, telle que le mammifère est humain.

9. Utilisation d'un polypeptide isolé codé par un gène suppresseur de tumeur associé au syndrome de carcinome basocellulaire naevoïde (NBCCS) et à différents cancers, notamment à divers carcinomes basocellulaires sporadiques (BCCs), pour la fabrication d'un médicament destiné à atténuer un symptôme du syndrome de carcinome basocellulaire naevoïde, ou d'un carcinome basocellulaire ou des kératoses solaires chez un mammifère, dans laquelle le polypeptide a la séquence d'acides aminés selon la SEQ ID N° 60.

10. Utilisation d'un acide nucléique isolé codant pour un gène suppresseur de tumeur associé au syndrome de carcinome basocellulaire naevoïde (NBCCS) et à différents cancers, notamment divers carcinomes basocellulaires sporadiques (BCCs), pour la fabrication d'un médicament destiné à atténuer un symptôme du syndrome de carcinome basocellulaire naevoïde, ou d'un carcinome basocellulaire ou des kératoses solaires chez un mammifère, dans laquelle l'acide nucléique comprend une région de codage codant pour un polypeptide ayant la séquence SEQ ID N° 60.

11. Utilisation selon la revendication 10, dans laquelle ledit acide nucléique a la séquence de nucléotides 442 à 4329 de la séquence SEQ ID N° 1.

12. Utilisation selon l'une des revendications 9 à 11, dans laquelle le mammifère est humain.
